# EUROPÄISCHE PATENTSCHRIFT

(11) **EP 3 259 330 B1**
(45) Veröffentlichungstag und Bekanntmachung des Hinweises auf die Patenterteilung: **14.11.2018**
(21) Anmeldenummer: 16701089.1
(22) Anmeldetag: 19.01.2016
(51) Int. Cl.: C09K 11/06, C07D 493/00, C07D 495/00, C07D 497/00, C07D 517/00, H01L 51/00, H01L 51/50, C07C 211/43, C07D 333/76, C07C 211/54, C07D 307/91, C07D 307/94, C07D 327/08, C07D 333/78, C07D 339/08, C07D 405/04, C07D 405/12, C07D 407/12, C07D 411/04

(54) **MATERIALIEN AUF BASIS VON SPIROBIFLUORENDERIVATEN FÜR ELEKTRONISCHE VORRICHTUNGEN**
SPIROBIFLUORENE DERIVATIVE-BASED MATERIALS FOR ELECTRONIC DEVICES
MATÉRIAUX À BASE DE DÉRIVÉS DE SPIROBIFLUORÈNE POUR DISPOSITIFS ÉLECTRONIQUES

(30) Priorität: 16.02.2015 EP 15000455
(43) Veröffentlichungstag der Anmeldung: 27.12.2017
(73) Patentinhaber: Merck Patent GmbH, 64293 Darmstadt (DE)
(72) Erfinder: PFISTER, Jochen, 74342 Seeheim-Jugenheim (DE); MUJICA-FERNAUD, Teresa, 64293 Darmstadt (DE); MONTENEGRO, Elvira, 69469 Weinheim (DE)
(86) Internationale Anmeldenummer: PCT/EP2016/000084
(87) Internationale Veröffentlichungsnummer: WO 2016/131521

(56) Entgegenhaltungen:
- EP-A1- 2 799 515
- WO-A1-2012/141229
- WO-A1-2014/010910
- WO-A1-2015/012618
- WO-A2-2014/058232
- US-A1- 2013 334 518

## Beschreibung

Die vorliegenden Anmeldung betrifft ein Spirobifluoren-Derivat einer im Folgenden definierten Formel (I), welches sich zur Verwendung in elektronischen Vorrichtungen, insbesondere organischen Elektrolumineszenzvorrichtungen (OLEDs) eignet.

Unter elektronischen Vorrichtungen im Sinne dieser Anmeldung werden sogenannte organische elektronische Vorrichtungen verstanden (organic electronic devices), welche organische Halbleitermaterialien als Funktionsmaterialien enthalten. Insbesondere werden darunter OLEDs verstanden.

Der Aufbau von OLEDs, in denen organische Verbindungen als funktionelle Materialien eingesetzt werden, ist beispielsweise in US 4539507, US 5151629, EP 0676461 und WO 98/27136 beschrieben. Allgemein werden unter der Bezeichnung OLEDs elektronische Vorrichtungen verstanden, welche eine oder mehrere Schichten enthaltend organische Verbindungen aufweisen und unter Anlegen von elektrischer Spannung Licht emittieren.

Bei elektronischen Vorrichtungen, insbesondere OLEDs, besteht großes Interesse an der Verbesserung der Leistungsdaten, insbesondere Lebensdauer, Effizienz und Betriebsspannung. In diesen Punkten konnte noch keine vollständig zufriedenstellende Lösung gefunden werden.

Einen großen Einfluss auf die Leistungsdaten von elektronischen Vorrichtungen haben Schichten mit lochtransportierender Funktion, wie beispielsweise lochinjizierende Schichten, Lochtransportschichten, Elektronenblockierschichten und auch emittierende Schichten. Zur Verwendung in diesen Schichten werden kontinuierlich neue Materialien mit lochtransportierenden Eigenschaften gesucht.

Es ist im Stand der Technik bekannt, Triarylamine als Materialien mit lochtransportierenden Eigenschaften in diesen Schichten einzusetzen. Die Triarylamine können Mono-Triarylamine darstellen, wie beispielsweise in JP 1995/053955, WO 2006/123667 und JP 2010/222268 beschrieben, oder Bis- oder andere Oligoamine darstellen, wie beispielsweise in US 7504163 oder US 2005/0184657 beschrieben. Bekannte Beispiele für Triarylamin-Verbindungen als Materialien mit lochtransportierenden Eigenschaften für OLEDs sind unter anderem Tris-p-biphenyl-amin, N,N'-Di-1-naphthyl-N,N'-diphenyl-1,1'-biphenyl-4,4'-diamin (NPB) und 4,4',4"-Tris-(3-methylphenylphenylamino)triphenylamin (MTDATA). Weiterhin im Stand der Technik bekannt sind hierfür Spirobifluoren-Arylaminoverbindungen, beispielsweise wie in WO 2012/034627 und WO 2013/120577 offenbart.

Ebenfalls im Stand der Technik bekannt für diese Verwendung sind Spirobifluoren-Derivate, die eine Benzofuran- oder Benzothiophen-Einheit in bestimmter Position ankondensiert an das Spirobifluoren-Grundgerüst aufweisen, und die eine oder mehrere Arylaminogruppen am Spirobifluoren gebunden aufweisen (WO 2013/100467).

Im Rahmen von Untersuchungen zu neuartigen Materialien zur Verwendung in OLEDs wurde nun überraschend gefunden, dass sich Verbindungen, die sich von den oben genannten Verbindungen dadurch unterscheiden, dass sie eine Benzofuran- oder Benzothiopheneinheit in einer anderen Position ankondensiert an das Spirobifluoren-Grundgerüst aufweisen, hervorragend zur Verwendung in OLEDs eignen, insbesondere als Materialien mit lochtransportierender Funktion. Insbesondere sind sie den oben genannten Verbindungen bezüglich ihrer Leistungsdaten bei der Verwendung in OLEDs überlegen, ganz besonders bei der Lebensdauer, der Betriebsspannung und der Quanteneffizienz der OLEDs. Die gefundenen neuen Verbindungen weisen weiterhin eine oder mehrere Eigenschaften gewählt aus sehr guten lochleitenden Eigenschaften, sehr guten elektronenblockierenden Eigenschaften, hoher Glasübergangstemperatur, hoher Oxidationsstabilität, guter Löslichkeit und hoher Temperaturstabilität auf.

Gegenstand der vorliegenden Erfindung ist daher eine Verbindung der Formel (I) 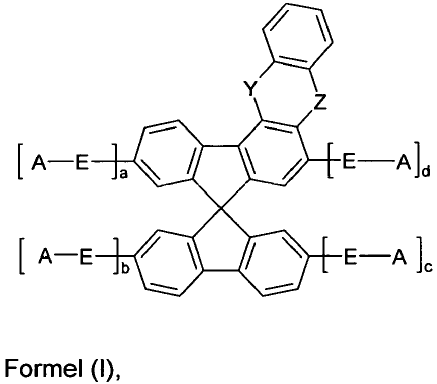 die an einer oder mehreren unsubstituiert dargestellten Positionen an der Grundstruktur gemäß Formel (I) mit je einem Rest R¹ substituiert sein kann; und
die die folgenden Definitionen der Variablen aufweist:
- Y: ist gewählt aus einer Einfachbindung, O, S oder Se;
- Z: ist gewählt aus O, S oder Se;
- E: ist bei jedem Auftreten gleich oder verschieden eine Einfachbindung oder ein aromatisches oder heteroaromatisches Ringsystem mit 6 bis 30 aromatischen Ringatomen, das mit einem oder mehreren Resten R² substituiert sein kann;
- A: ist bei jedem Auftreten gleich oder verschieden eine Gruppe der Formel (A1), (A2) oder (A3), die über die mit # markierte Bindung gebunden ist;

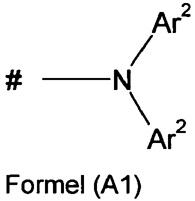

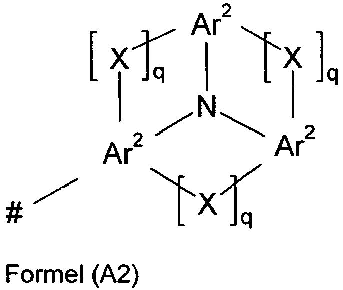

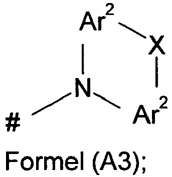

- Ar²: ist bei jedem Auftreten gleich oder verschieden ein aromatisches oder heteroaromatisches Ringsystem mit 6 bis 30 aromatischen Ringatomen, das mit einem oder mehreren Resten R² substituiert sein kann;
- X: ist bei jedem Auftreten gleich oder verschieden eine Einfachbindung oder eine Gruppe gewählt aus BR², C(R²)₂, Si(R²)₂, C=O, O, S, S=O, SO₂, NR², PR² oder P(=O)R²;
- R¹: ist bei jedem Auftreten gleich oder verschieden gewählt aus H, D, F, C(=O)R³, CN, Si(R³)₃, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können;
- R²: ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können;
- R³: ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
- R⁴: ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkylgruppen, aromatischen Ringsysteme und heteroaromatischen Ringsysteme mit F oder CN substituiert sein können;
- q: ist bei jedem Auftreten gleich oder verschieden 0 oder 1, wobei mindestens ein q in Formel (A2) gleich 1 ist;
a, b, c und d sind bei jedem Auftreten gleich oder verschieden 0 oder 1, wobei mindestens einer der Indices a, b, c und d gleich 1 ist, und wobei für den Fall, dass einer oder mehrere der Indices a, b, c und d gleich 0 sind, an der betreffenden Position eine Gruppe R¹ gebunden ist.

Eine Arylgruppe im Sinne dieser Erfindung enthält 6 bis 40 aromatische Ringatome, von denen keines ein Heteroatom darstellt. Unter einer Arylgruppe im Sinne dieser Erfindung wird entweder ein einfacher aromatischer Cyclus, also Benzol, oder ein kondensierter aromatischer Polycyclus, beispielsweise Naphthalin, Phenanthren oder Anthracen, verstanden. Ein kondensierter aromatischer Polycyclus besteht im Sinne der vorliegenden Anmeldung aus zwei oder mehr miteinander kondensierten einfachen aromatischen Cyclen. Unter Kondensation zwischen Cyclen ist dabei zu verstehen, dass die Cyclen mindestens eine Kante miteinander teilen.

Eine Heteroarylgruppe im Sinne dieser Erfindung enthält 5 bis 40 aromatische Ringatome, von denen mindestens eines ein Heteroatom darstellt. Die Heteroatome der Heteroarylgruppe sind bevorzugt ausgewählt aus N, O und S. Unter einer Heteroarylgruppe im Sinne dieser Erfindung wird entweder ein einfacher heteroaromatischer Cyclus, beispielsweise Pyridin, Pyrimidin oder Thiophen, oder ein kondensierter heteroaromatischer Polycyclus, beispielsweise Chinolin oder Carbazol, verstanden. Ein kondensierter heteroaromatischer Polycyclus besteht im Sinne der vorliegenden Anmeldung aus zwei oder mehr miteinander kondensierten einfachen heteroaromatischen Cyclen. Unter Kondensation zwischen Cyclen ist dabei zu verstehen, dass die Cyclen mindestens eine Kante miteinander teilen.

Ein aromatisches Ringsystem im Sinne dieser Erfindung enthält 6 bis 40 C-Atome im Ringsystem und umfasst keine Heteroatome als aromatische Ringatome. Ein aromatisches Ringsystem im Sinne dieser Erfindung enthält daher keine Heteroarylgruppen. Unter einem aromatischen Ringsystem im Sinne dieser Erfindung soll ein System verstanden werden, das nicht notwendigerweise nur Arylgruppen enthält, sondern in dem auch mehrere Arylgruppen durch eine Einfachbindung oder durch eine nicht-aromatische Einheit, wie beispielsweise ein oder mehrere wahlweise substituierte C-, Si-, N-, O- oder S-Atome, verbunden sein können. Dabei umfasst die nicht-aromatische Einheit bevorzugt weniger als 10 % der von H verschiedenen Atome, bezogen auf die Gesamtzahl der von H verschiedenen Atome des Systems. So sollen beispielsweise auch Systeme wie 9,9'-Spirobifluoren, 9,9'-Diarylfluoren, Triarylamin, Diarylether und Stilben als aromatische Ringsysteme im Sinne dieser Erfindung verstanden werden, und ebenso Systeme, in denen zwei oder mehr Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Arylgruppen über Einfachbindungen miteinander verknüpft sind, als aromatische Ringsysteme im Sinne dieser Erfindung verstanden, wie beispielsweise Systeme wie Biphenyl und Terphenyl.

Ein heteroaromatisches Ringsystem im Sinne dieser Erfindung enthält 5 bis 40 aromatische Ringatome, von denen mindestens eines ein Heteroatom darstellt. Die Heteroatome des heteroaromatischen Ringsystems sind bevorzugt ausgewählt aus N, O und/oder S. Ein heteroaromatisches Ringsystem entspricht der oben genannten Definition eines aromatischen Ringsystems, weist jedoch mindestens ein Heteroatom als eines der aromatischen Ringatome auf. Es unterscheidet sich dadurch von einem aromatischen Ringsystem im Sinne der Definition der vorliegenden Anmeldung, welches gemäß dieser Definition kein Heteroatom als aromatisches Ringatom enthalten kann.

Unter einer Aryl- oder Heteroarylgruppe werden insbesondere Gruppen verstanden, welche abgeleitet sind von Benzol, Naphthalin, Anthracen, Phenanthren, Pyren, Dihydropyren, Chrysen, Perylen, Triphenylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einem aromatischen Ringsystem mit 6 bis 40 aromatischen Ringatomen oder einem heteroaromatischen Ringsystem mit 5 bis 40 aromatischen Ringatomen werden insbesondere Gruppen verstanden, die abgeleitet sind von den oben unter Arylgruppen und Heteroarylgruppen genannten Gruppen sowie von Biphenyl, Terphenyl, Quaterphenyl, Fluoren, Spirobifluoren, Dihydrophenanthren, Dihydropyren, Tetrahydropyren, Indenofluoren, Truxen, Isotruxen, Spirotruxen, Spiroisotruxen, Indenocarbazol, oder von Kombinationen dieser Gruppen.

Im Rahmen der vorliegenden Erfindung werden unter einer geradkettigen Alkylgruppe mit 1 bis 20 C-Atomen bzw. einer verzweigten oder cyclischen Alkylgruppe mit 3 bis 20 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neo-Pentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden.

Unter einer Alkoxy- oder Thioalkylgruppe mit 1 bis 20 C-Atomen, in der auch einzelne H-Atome oder CH₂-Gruppen durch die oben bei der Definition der Reste genannten Gruppen substituiert sein können, werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Es ist erfindungsgemäß bevorzugt, dass Y gewählt ist aus einer Einfachbindung, O oder S. Es ist weiterhin erfindungsgemäß bevorzugt, dass Z gewählt ist aus O oder S. Es ist besonders bevorzugt, dass Y eine Einfachbindung ist, und dass Z gewählt ist aus O oder S. Gemäß einer weiteren Ausführungsform der Erfindung ist es bevorzugt, dass Y gewählt ist aus O oder S, und dass Z gewählt ist aus O oder S.

Es ist weiterhin bevorzugt, dass E bei jedem Auftreten gleich oder verschieden gewählt ist aus einer Einfachbindung oder einer divalenten Gruppe abgeleitet von wahlweise mit Resten R² substituiertem Benzol, Biphenyl, Terphenyl, Fluoren, Spirobifluoren, Indenofluoren, Carbazol, Dibenzofuran, Dibenzothiophen, oder einer Kombination von zwei oder mehr dieser Gruppen, wobei nicht mehr als 30 aromatische Ringatome in der Gruppe E vorliegen.

Gruppen E sind bevorzugt gewählt aus einer Einfachbindung oder aus Gruppen der folgenden Formeln: 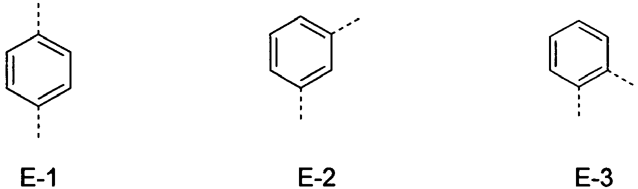 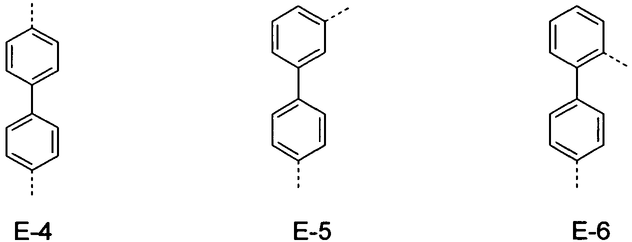 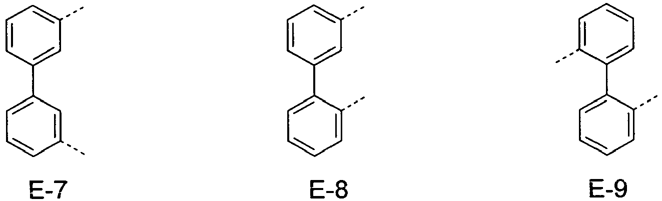 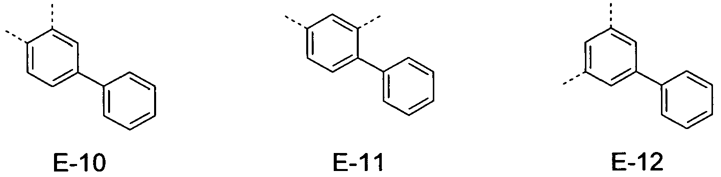 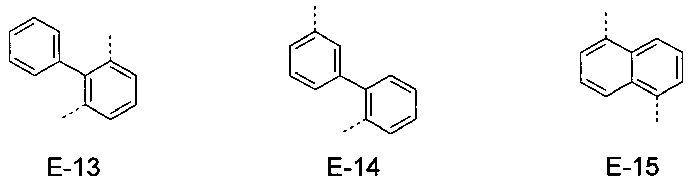 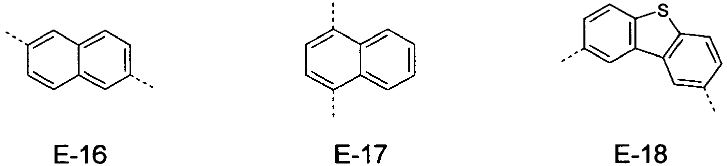 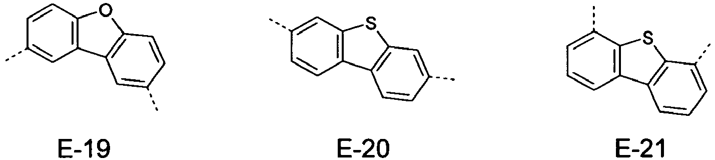 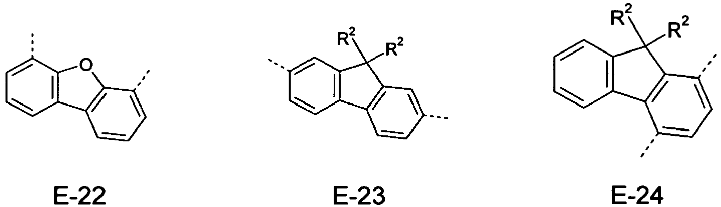 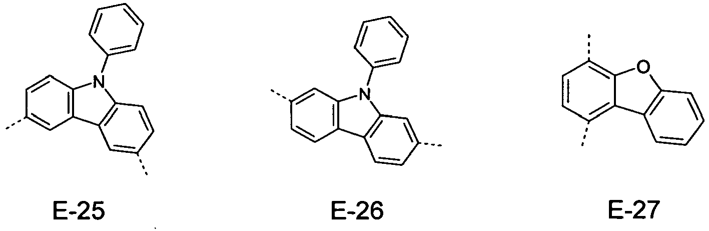 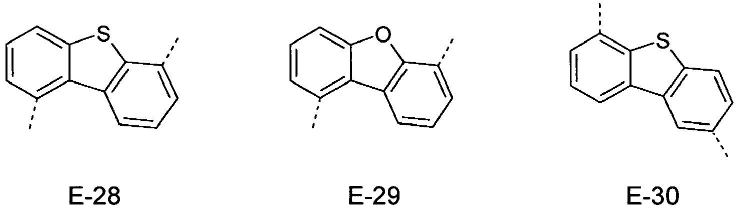 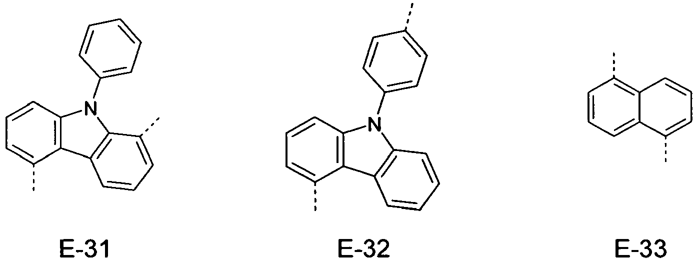 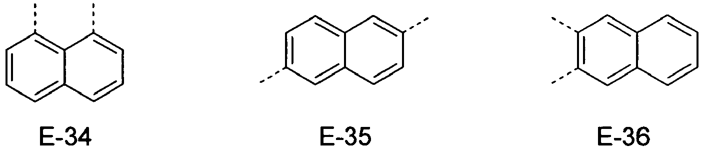 wobei die gestrichelten Bindungen die Bindungen an den Rest der Formel darstellen und die Gruppen an den freien Positionen durch einen oder mehrere Reste R² substituiert sein können, bevorzugt an den freien Positionen aber unsubstituiert sind.

Dabei steht R² in den Gruppen der Formeln (E-23) und (E-24) bevorzugt gleich oder verschieden für eine Alkylgruppe mit 1 bis 10 C-Atomen, insbesondere für Methyl, oder für eine Phenylgruppe, die mit einem oder mehreren Resten R³ substituiert sein kann und bevorzugt unsubstituiert ist. Zwei Alkylgruppen R² können dabei auch unter Ausbildung einer Spiro-Gruppe einen Ring bilden, bevorzugt einen Cyclohexylring oder einen Cyclopentylring.

Es ist bevorzugt, dass A bei jedem Auftreten gleich oder verschieden eine Gruppe der Formel (A-1) oder (A-3) ist, besonders bevorzugt eine Gruppe der Formel (A-1) ist.

Eine bevorzugte Ausführungsform der Gruppe der Formel (A-3) ist eine Gruppe der folgenden Formel (A-3-1) 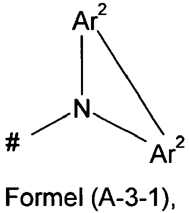 wobei die Gruppe Ar² wie obenstehend definiert ist, und bevorzugt definiert ist gemäß ihren bevorzugten Ausführungsformen.

Bevorzugte Ausführungsformen der Gruppen der Formel (A-3-1) entsprechen den folgenden Formeln 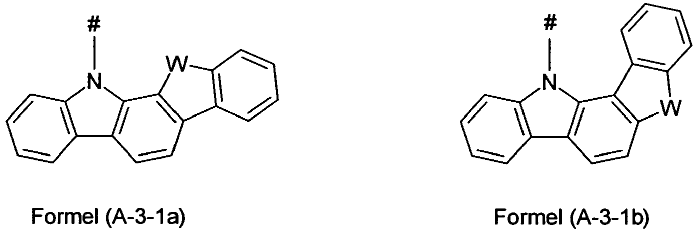 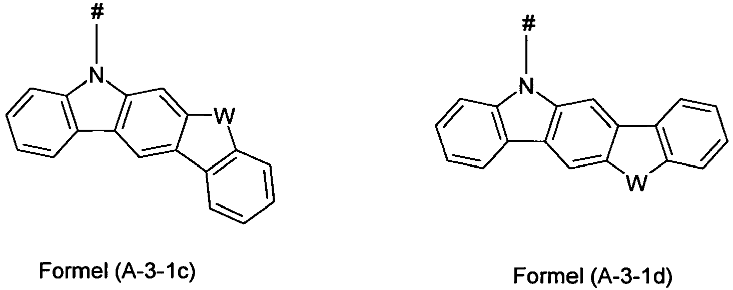 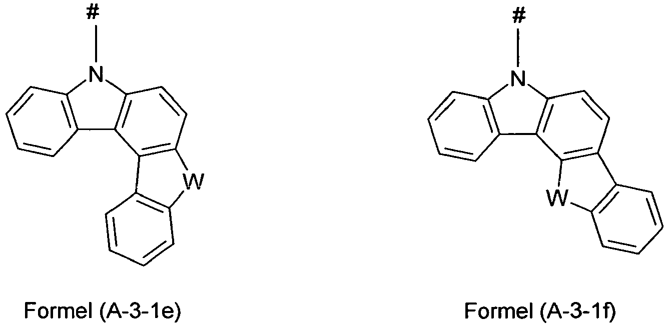 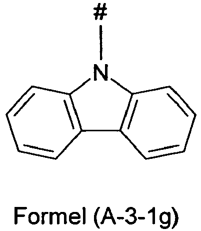 wobei die Gruppen an den freien Positionen je mit einem Rest R² substituiert sein können, und
wobei W bei jedem Auftreten gleich oder verschieden gewählt ist aus BR², C(R²)₂, Si(R²)₂, C=O, O, S, S=O, SO₂, NR², PR² oder P(=O)R², und bevorzugt gewählt ist aus C(R²)₂, O, S und NR², und besonders bevorzugt gleich C(R²)₂ ist.

Besonders bevorzugt unter den Gruppen der Formeln (A-3-1a) bis (A-3-1g) sind die Gruppen (A-3-1c) und (A-3-1g).

Es ist bevorzugt, dass Ar² bei jedem Auftreten gleich oder verschieden gewählt ist aus einem aromatischen oder heteroaromatischen Ringsystem mit 6 bis 25 aromatischen Ringatomen, das mit einem oder mehreren Resten R² substituiert sein kann. Besonders bevorzugt sind Phenyl, Biphenyl, Terphenyl, Fluorenyl, Spirobifluorenyl, Indenofluorenyl, Naphthyl, Phenanthrenyl, Furanyl, Benzofuranyl, Dibenzofuranyl, Thiophenyl, Benzothiophenyl, Dibenzothiophenyl, Carbazolyl, Indolocarbazolyl und Indenocarbazolyl, welche jeweils mit einem oder mehreren Resten R² substituiert sein können.

Gruppen Ar² sind bevorzugt gleich oder verschieden bei jedem Auftreten gewählt aus Gruppen der folgenden Formeln: 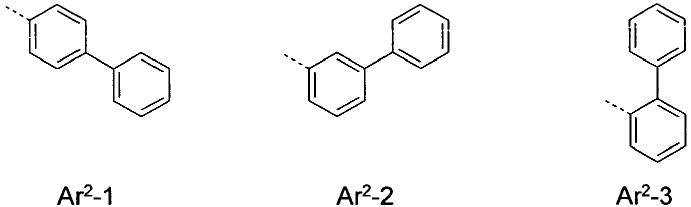 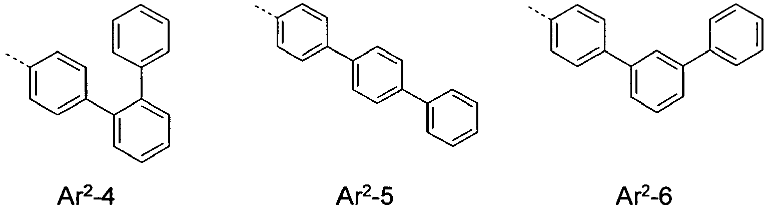 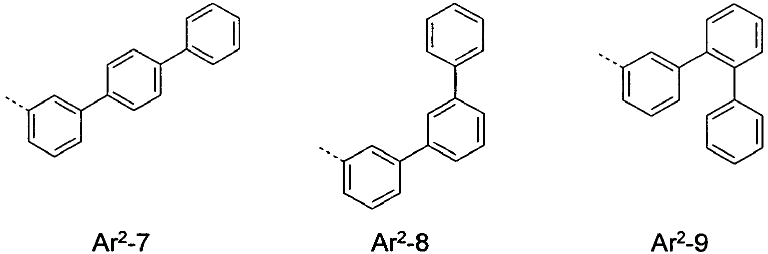 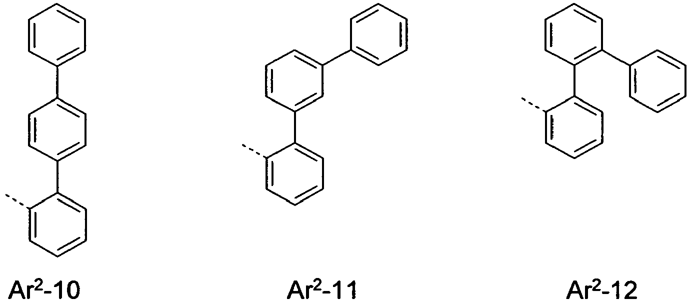 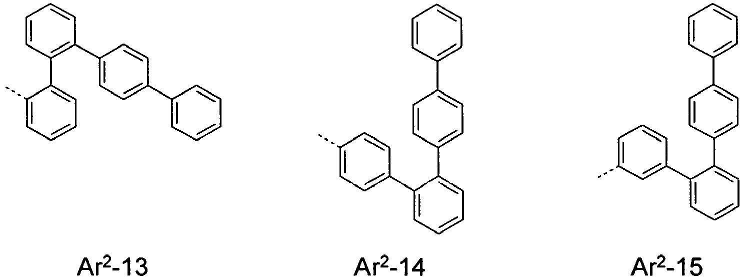 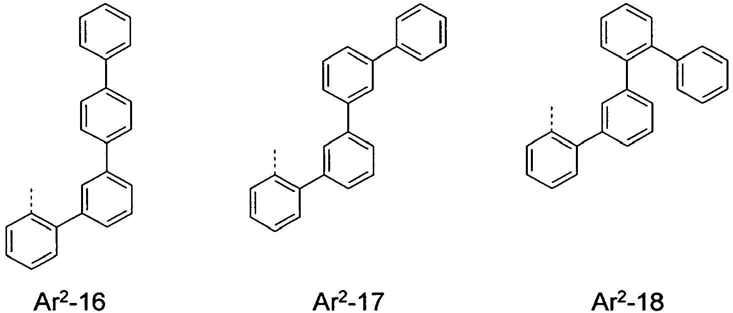 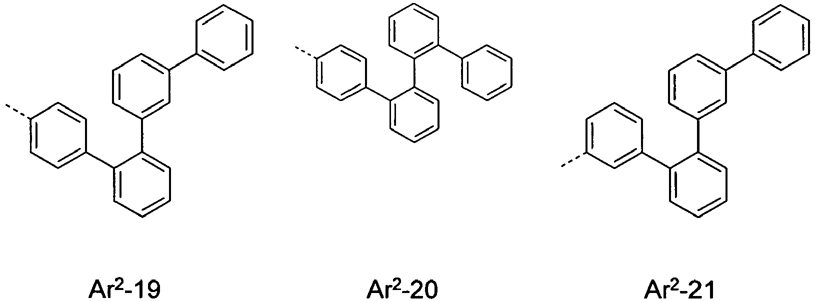 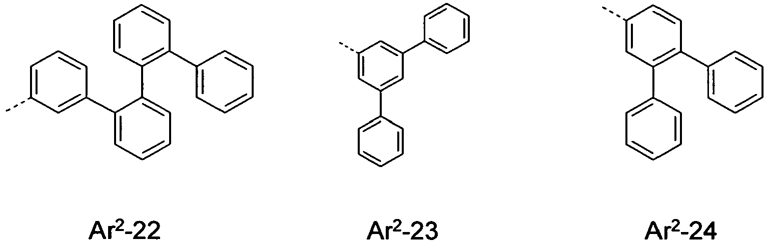 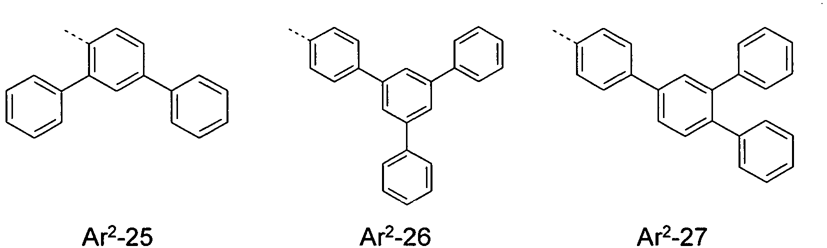 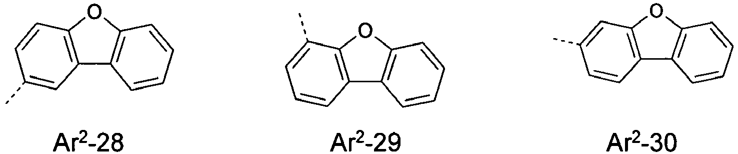 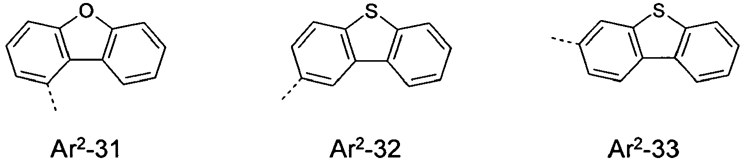 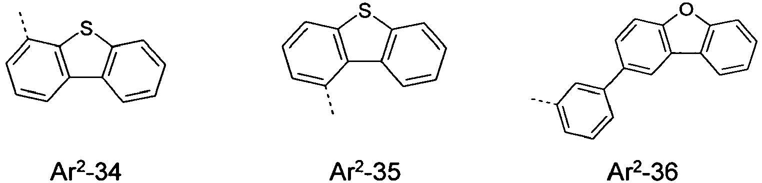 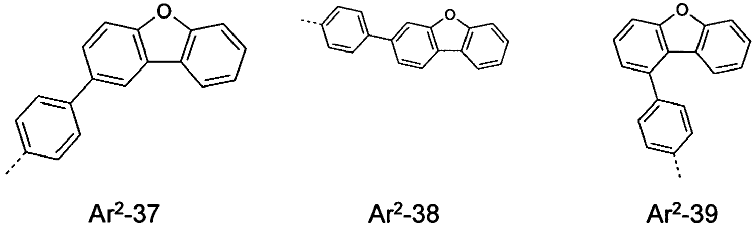 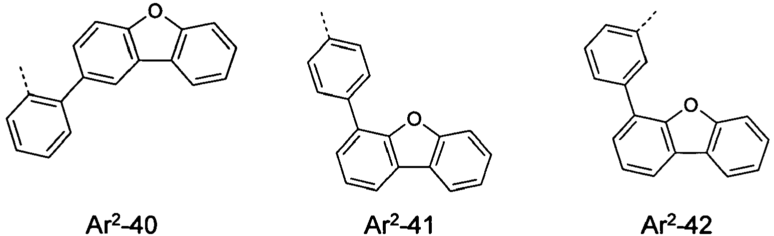 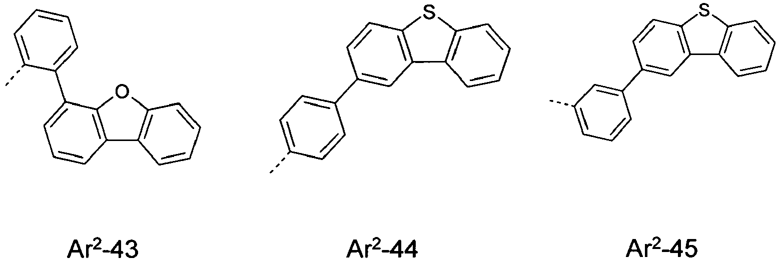 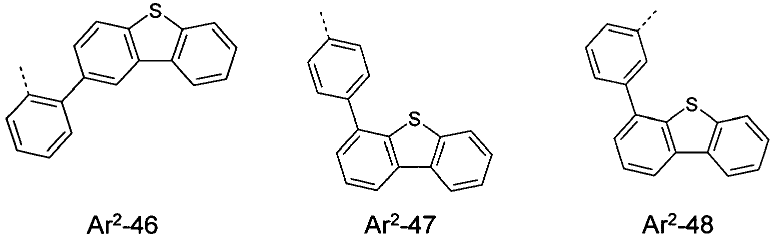 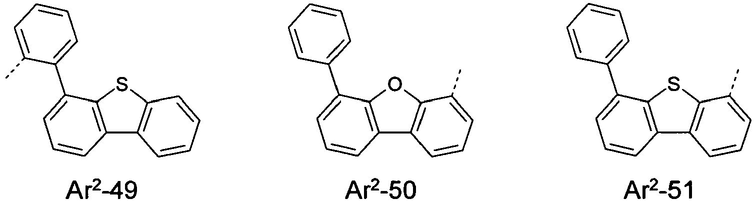 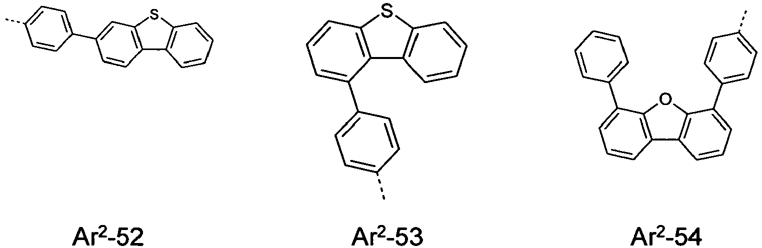 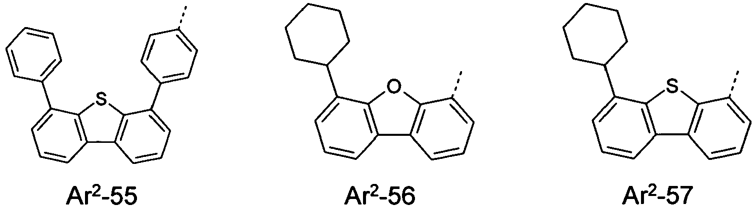 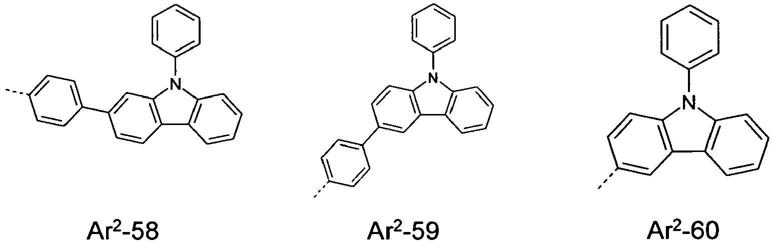 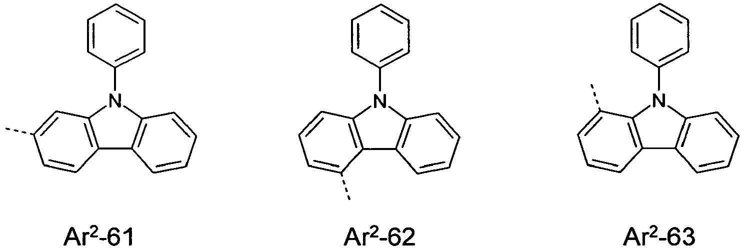 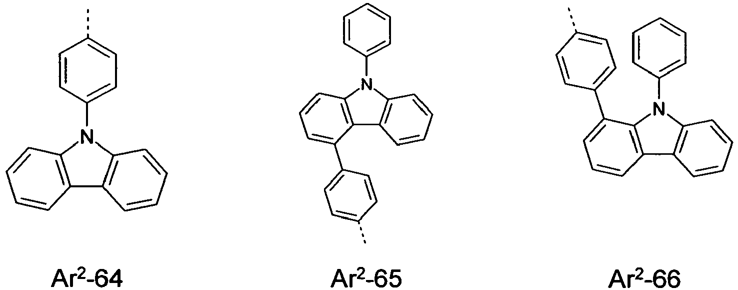 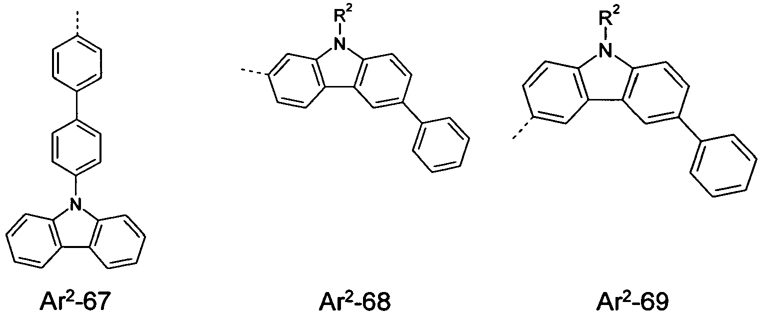 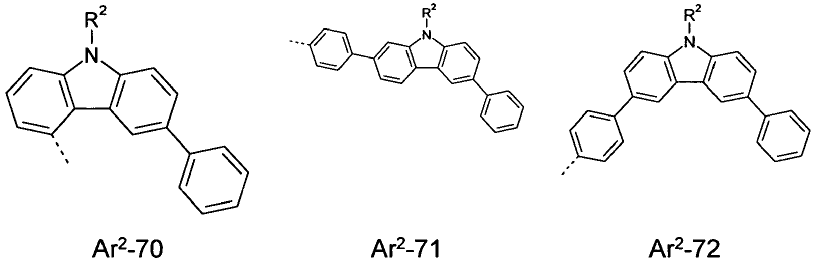 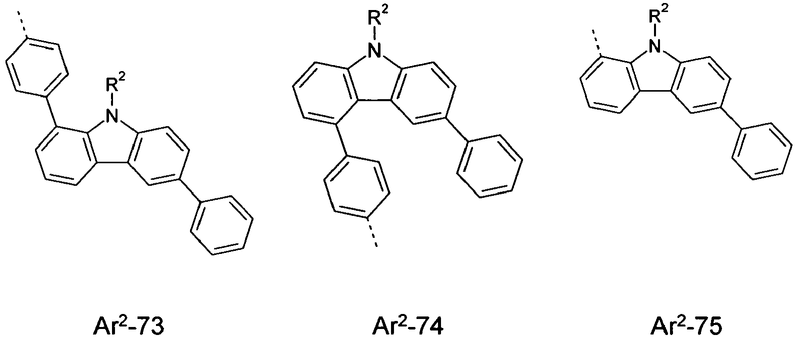 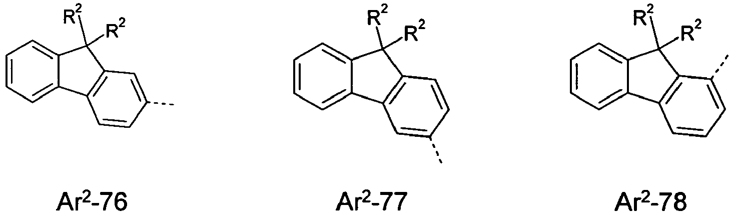 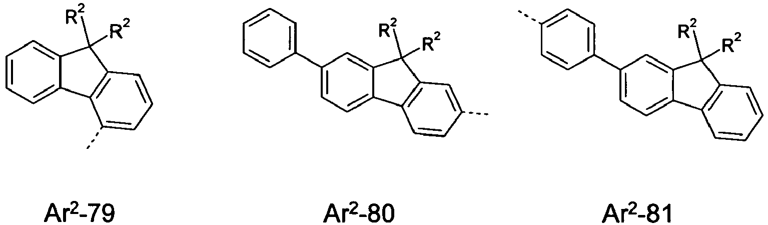 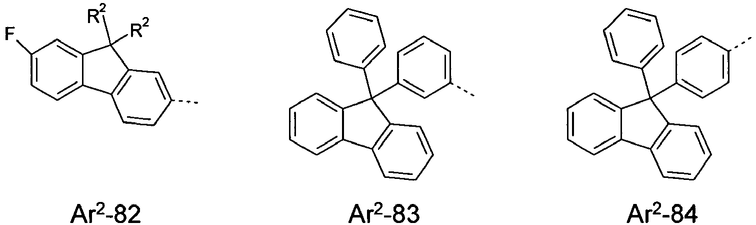 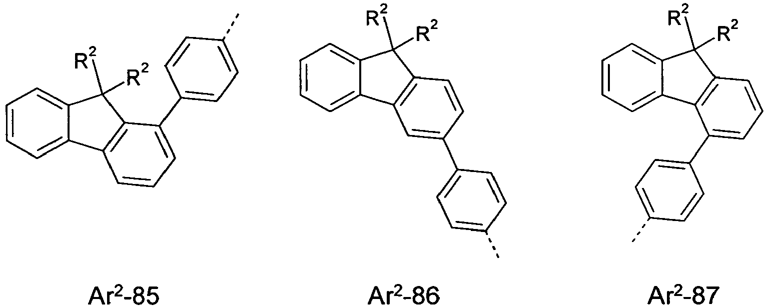 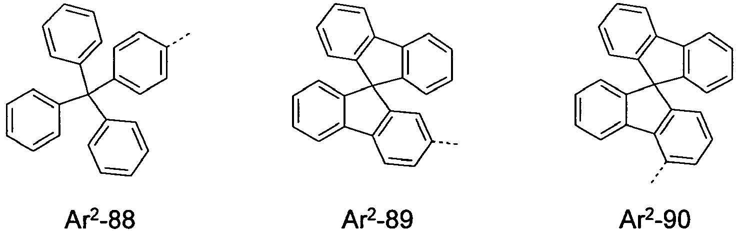 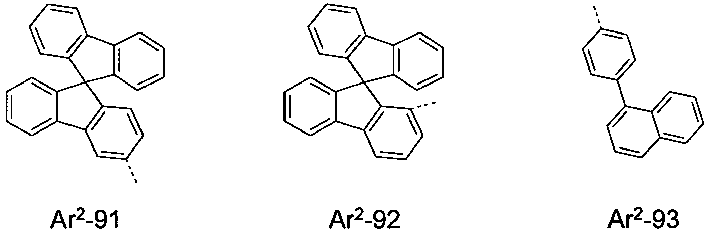 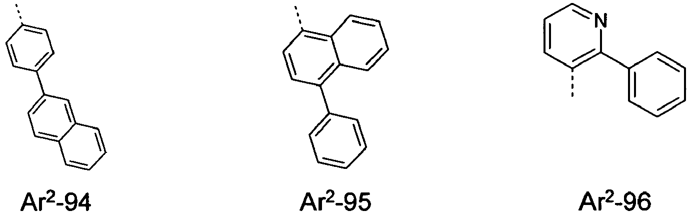 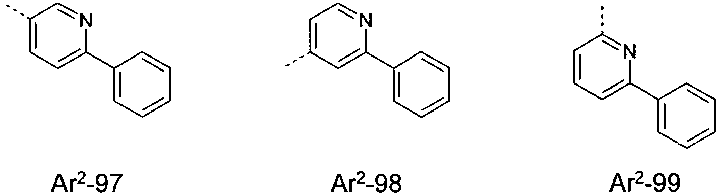 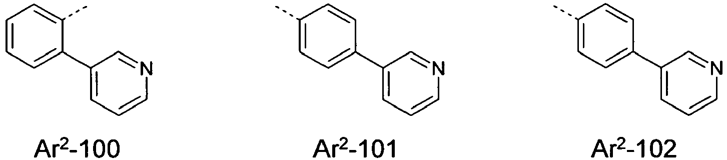 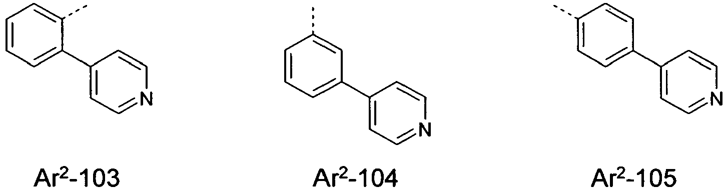 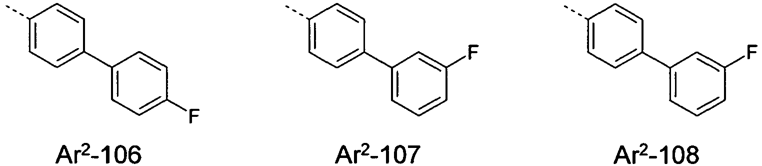 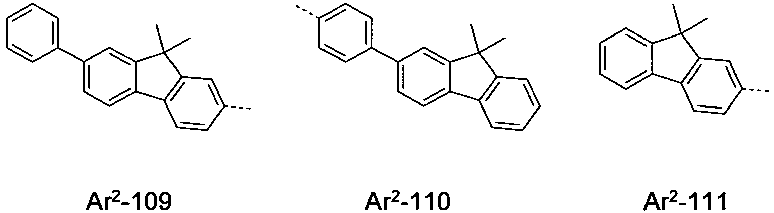 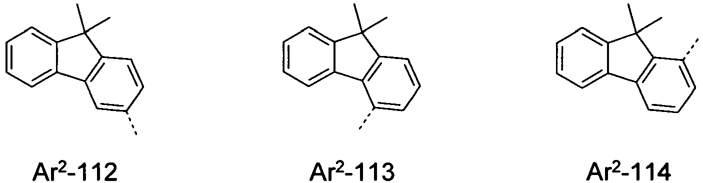 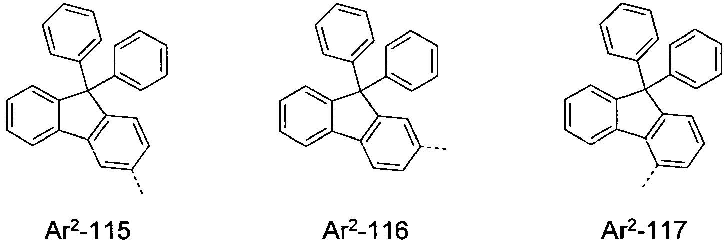 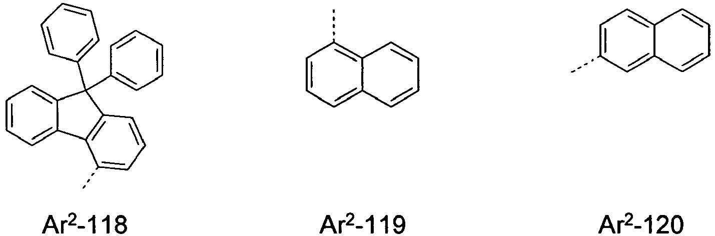 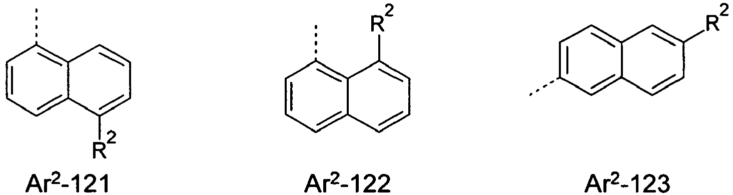 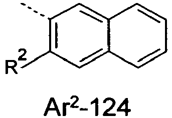 wobei die gestrichelte Bindung die Bindung an den Stickstoff darstellt und die Gruppen an den freien Positionen durch einen oder mehrere Reste R² substituiert sein können, bevorzugt an den freien Positionen aber unsubstituiert sind.

Dabei steht R² in den Gruppen der Formeln (Ar²-68) bis (Ar²-82) und (Ar²-85) bis (Ar²-87) bevorzugt gleich oder verschieden für eine Alkylgruppe mit 1 bis 10 C-Atomen, insbesondere für Methyl, oder eine Phenylgruppe, die mit einem oder mehreren Resten R³ substituiert sein kann und bevorzugt unsubstituiert ist. Zwei Alkylgruppen R² können dabei auch unter Ausbildung einer Spiro-Gruppe einen Ring bilden, bevorzugt einen Cyclohexylring oder einen Cyclopentylring.

Es ist bevorzugt, dass X bei jedem Auftreten gleich oder verschieden gewählt ist aus einer Einfachbindung oder einer Gruppe gewählt aus C(R²)₂, C=O, O, S und NR²; besonders bevorzugt ist X eine Einfachbindung.

Bevorzugt ist R¹ bei jedem Auftreten gleich oder verschieden H, D, F, CN, Si(R³)₃, eine geradkettige Alkyl- oder Alkoxygruppe mit 1 bis 10 C-Atomen, eine verzweigte oder cyclische Alkyl- oder Alkoxygruppe mit 3 bis 10 C-Atomen, oder ein aromatisches oder heteroaromatisches Ringsystem mit 6 bis 30 aromatischen Ringatomen, wobei zwei oder mehr Reste R¹ miteinander verknüpft sein können und einen Ring bilden können, wobei die genannten Alkyl- und Alkoxygruppen und die genannten aromatischen und heteroaromatischen Ringsysteme jeweils mit einem oder mehreren Resten R³ substituiert sein können, und wobei in den genannten Alkyl- und Alkoxygruppen eine oder mehrere CH₂-Gruppen durch -C=C-, -R³C=CR³-, Si(R³)₂, C=O, C=NR³, -NR³-, -O-, -S-, -C(=O)O- oder -C(=O)NR³-ersetzt sein können. Besonders bevorzugt ist R¹ bei jedem Auftreten gleich oder verschieden H, D, F, CN, eine geradkettige Alkylgruppe mit 1 bis 10 C-Atomen oder eine verzweigte oder cyclische Alkylgruppe mit 3 bis 10 C-Atomen, oder ein aromatisches oder heteroaromatisches Ringsystem mit 6 bis 25 aromatischen Ringatomen, wobei die genannten Alkyl- und Alkoxygruppen und die genannten aromatischen oder heteroaromatischen Ringsysteme jeweils mit einem oder mehreren Resten R³ substituiert sein können. Ganz besonders bevorzugt ist R¹ bei jedem Auftreten gleich oder verschieden H, F, CN, Methyl, tert-Butyl, Phenyl, Biphenyl, Dibenzofuran, Dibenzothiophen oder Carbazol, nochmals stärker bevorzugt gleich H.

Bevorzugt ist R² bei jedem Auftreten gleich oder verschieden H, D, F, CN, Si(R³)₃, eine geradkettige Alkyl- oder Alkoxygruppe mit 1 bis 10 C-Atomen, eine verzweigte oder cyclische Alkyl- oder Alkoxygruppe mit 3 bis 10 C-Atomen, oder ein aromatisches oder heteroaromatisches Ringsystem mit 6 bis 30 aromatischen Ringatomen, wobei zwei oder mehr Reste R² miteinander verknüpft sein können und einen Ring bilden können, wobei die genannten Alkyl- und Alkoxygruppen und die genannten aromatischen und heteroaromatischen Ringsysteme jeweils mit einem oder mehreren Resten R³ substituiert sein können, und wobei in den genannten Alkyl- und Alkoxygruppen eine oder mehrere CH₂-Gruppen durch -C=C-, -R³C=CR³-, Si(R³)₂, C=O, C=NR³, -NR³-, -O-, -S-, -C(=O)O- oder -C(=O)NR³- ersetzt sein können. Besonders bevorzugt ist R² bei jedem Auftreten gleich oder verschieden H, D, F, CN, eine geradkettige Alkylgruppe mit 1 bis 10 C-Atomen oder eine verzweigte oder cyclische Alkylgruppe mit 3 bis 10 C-Atomen, oder ein aromatisches oder heteroaromatisches Ringsystem mit 6 bis 25 aromatischen Ringatomen, wobei die genannten Alkyl- und Alkoxygruppen und die genannten aromatischen oder heteroaromatischen Ringsysteme jeweils mit einem oder mehreren Resten R³ substituiert sein können. Ganz besonders bevorzugt ist R² bei jedem Auftreten gleich oder verschieden H, F, CN, Methyl, tert-Butyl, Phenyl, Biphenyl, Dibenzofuran, Dibenzothiophen oder Carbazol.

Bevorzugt ist R³ bei jedem Auftreten gleich oder verschieden H, D, F, CN, Si(R⁴)₃, N(R⁴)₂, eine geradkettige Alkyl- oder Alkoxygruppe mit 1 bis 10 C-Atomen oder eine verzweigte oder cyclische Alkyl- oder Alkoxygruppe mit 3 bis 10 C-Atomen, oder ein aromatisches oder heteroaromatisches Ringsystem mit 5 bis 30 aromatischen Ringatomen, wobei zwei oder mehr Reste R³ miteinander verknüpft sein können und einen Ring bilden können, wobei die genannten Alkyl- und Alkyoxygruppen und die genannten aromatischen oder heteroaromatischen Ringsysteme jeweils mit einem oder mehreren Resten R⁴ substituiert sein können, und wobei in den genannten Alkyl- und Alkyoxygruppen eine oder mehrere CH₂-Gruppen durch -C≡C-, -R⁴C=CR⁴-, Si(R⁴)₂, C=O, C=NR⁴, -NR⁴-, -O-, -S-,-C(=O)O- oder -C(=O)NR⁴- ersetzt sein können. Besonders bevorzugt ist R³ bei jedem Auftreten gleich oder verschieden H, D, F, CN, N(R⁴)₂, eine geradkettige Alkylgruppe mit 1 bis 10 C-Atomen oder eine verzweigte oder cyclische Alkylgruppe mit 3 bis 10 C-Atomen, oder ein aromatisches oder heteroaromatisches Ringsystem mit 6 bis 25 aromatischen Ringatomen, wobei die genannten Alkyl- und Alkyoxygruppen und die genannten aromatischen oder heteroaromatischen Ringsysteme jeweils mit einem oder mehreren Resten R⁴ substituiert sein können.

Es ist erfindungsgemäß bevorzugt, dass genau einer der Indices a, b, c und d gleich 1 ist, und die anderen Indices gleich 0 sind; oder dass genau zwei der Indices a, b, c und d gleich 1 sind, und die anderen Indices gleich 0 sind.

Weiterhin ist es bevorzugt, dass der Index d gleich 0 ist, und dass einer oder mehrere, bevorzugt genau einer oder genau zwei der Indices a, b und c gleich 1 sind.

Weiterhin ist es bevorzugt, dass wenn Y eine Einfachbindung ist, und Z gleich O ist, und der Index a gleich 1 ist, mindestens einer der Indices b, c und d gleich 1 ist. Besonders bevorzugt ist es, dass wenn der Index a gleich 1 ist, mindestens einer der Indices b, c und d gleich 1 ist.

Eine bevorzugte Ausführungsform der Formel (I) entspricht der folgenden Formel 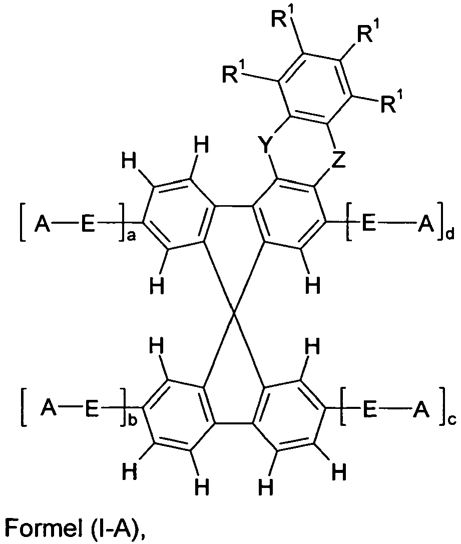 wobei die auftretenden Variablen definiert sind wie oben, und bevorzugt ihren bevorzugten Ausführungsformen entsprechen. An den mit "H" bezeichneten Positionen kann dabei nur Wasserstoff vorliegen. Bevorzugt ist in Formel (I-A) R¹ gleich Wasserstoff.

Auch in diesem Fall ist es bevorzugt, dass wenn Y eine Einfachbindung ist, und Z gleich O ist, und der Index a gleich 1 ist, mindestens einer der Indices b, c und d gleich 1 ist. Besonders bevorzugt ist es, dass wenn der Index a gleich 1 ist, mindestens einer der Indices b, c und d gleich 1 ist.

Bevorzugte Ausführungsformen der Formel (I) entsprechen den folgenden Formeln 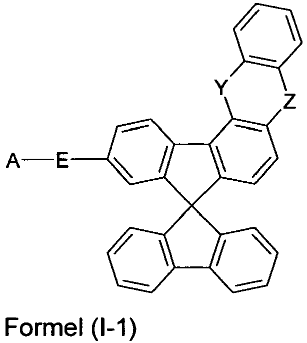 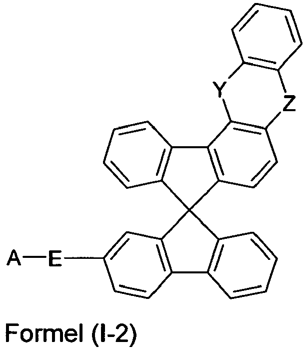 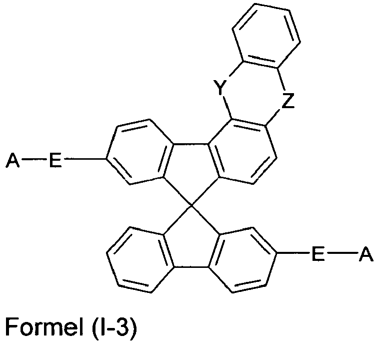 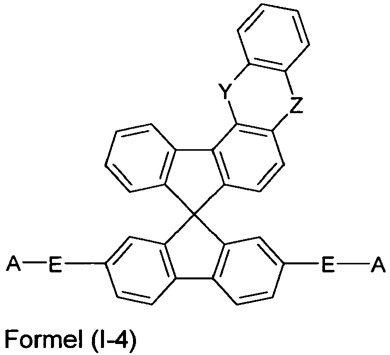 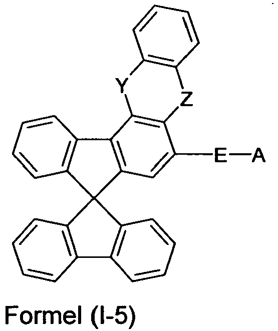 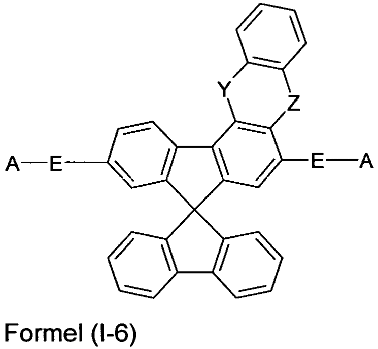 wobei die auftretenden Variablen wie oben definiert sind und bevorzugt ihren bevorzugten Ausführungsformen entsprechen, und wobei die Grundstruktur gemäß Formel (I-1) bis (I-6) an einer oder mehreren unsubstituiert dargestellten Positionen mit je einem Rest R¹ substituiert sein kann.

Für Formel (I-1) ist es bevorzugt, dass der Fall ausgeschlossen ist, dass Y eine Einfachbindung ist und gleichzeitig Z gleich O ist.

Unter den Formeln (I-1) bis (I-6) sind die Formeln (I-1) bis (I-4) bevorzugt, sind die Formeln (I-1) und (I-2) besonders bevorzugt, und ganz besonders bevorzugt ist die Formel (I-2).

Bevorzugte Ausführungsformen der Formeln (I-1) bis (I-6) entsprechen den folgenden Formeln 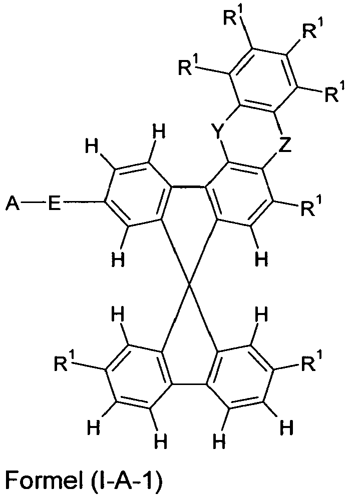 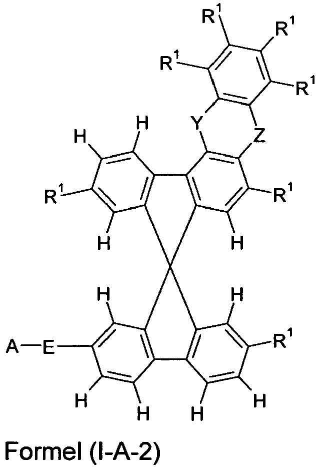 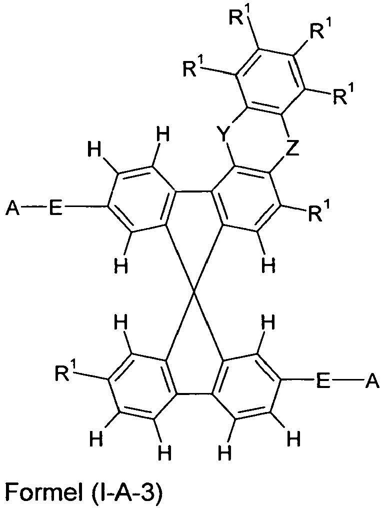 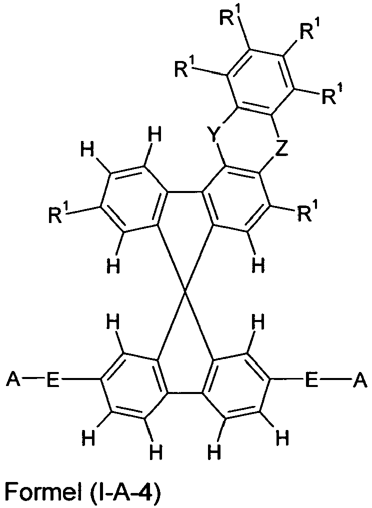 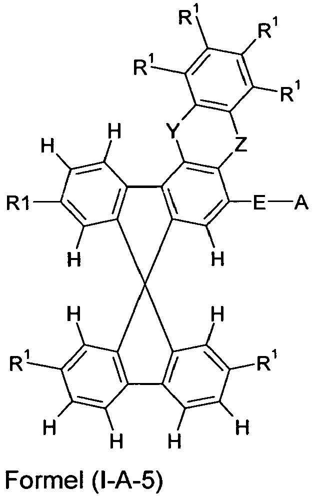 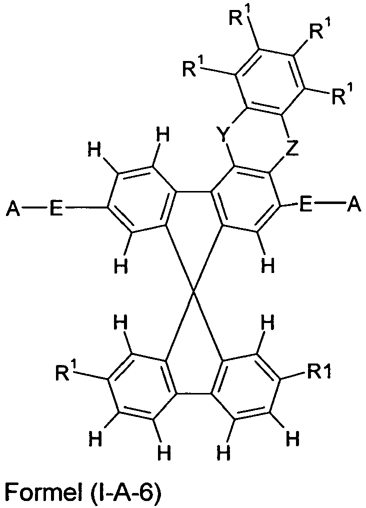 wobei die auftretenden Variablen definiert sind wie oben, und bevorzugt ihren bevorzugten Ausführungsformen entsprechen. An den mit "H" bezeichneten Positionen kann dabei nur Wasserstoff vorliegen. Bevorzugt ist in den Formeln (I-A-1) bis (I-A-6) R¹ gleich Wasserstoff.

Unter den Formeln (I-A-1) bis (I-A-6) sind die Formeln (I-A-1) bis (I-A-4) bevorzugt, die Formeln (I-A-1) und (I-A-2) besonders bevorzugt, und ganz besonders bevorzugt ist die Formel (I-A-2).

Für Formel (I-A-1) ist es bevorzugt, dass der Fall ausgeschlossen ist, dass Y eine Einfachbindung ist und gleichzeitig Z gleich O ist.

Weiterhin entsprechen bevorzugte Ausführungsformen der Formeln (I-1) bis (I-6) den folgenden Formeln 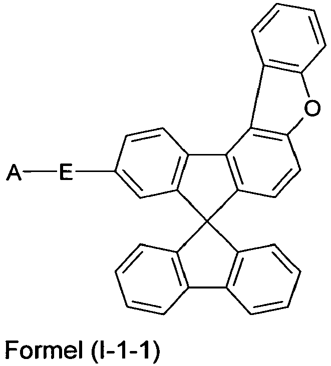 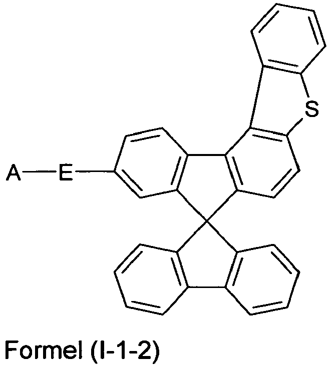 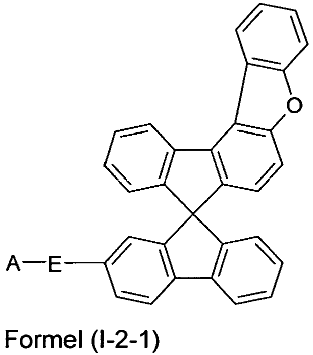 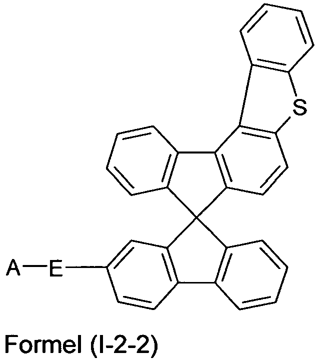 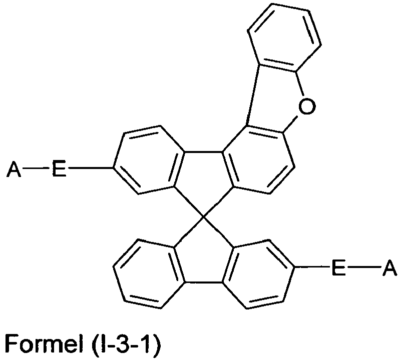 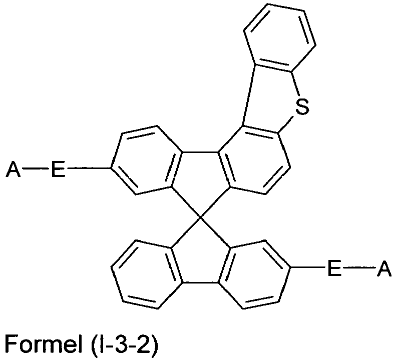 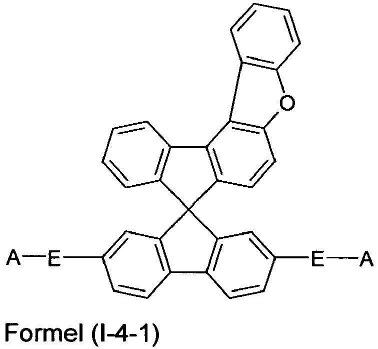 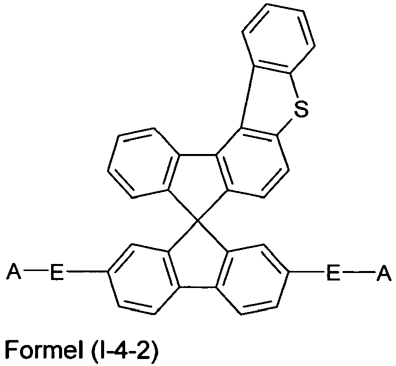 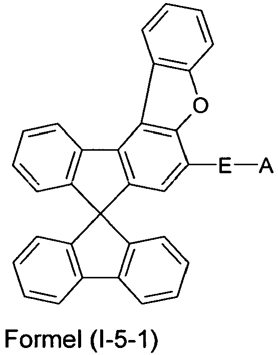 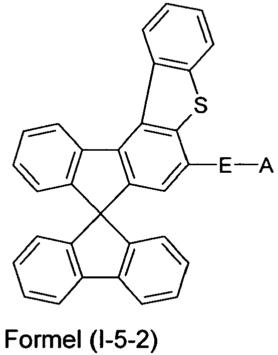 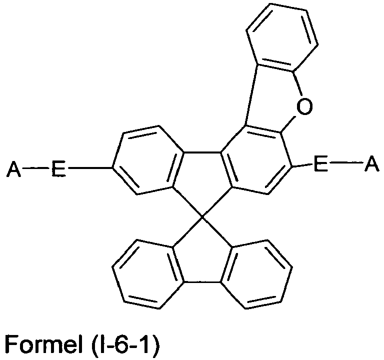 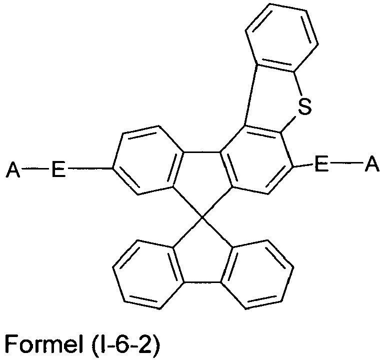 wobei die auftretenden Variablen wie oben definiert sind und bevorzugt ihren bevorzugten Ausführungsformen entsprechen, und wobei die Grundstruktur gemäß Formel (1-1-1) bis (I-6-2) an einer oder mehreren unsubstituiert dargestellten Positionen mit je einem Rest R¹ substituiert sein kann.

Unter den genannten Formeln sind die Formeln (I-1-1) bis (I-4-2) bevorzugt.

Ganz besonders bevorzugte Ausführungsformen der Verbindungen gemäß Formel (I) entsprechen einer der Formeln (I-A-1) bis (I-A-6) oder einer der Formeln (I-1-1) bis (I-6-2), wobei zusätzlich gilt:
Y ist, sofern vorhanden, gewählt aus einer Einfachbindung, O oder S;
Z ist, sofern vorhanden, gewählt aus O oder S;
E ist bei jedem Auftreten gleich oder verschieden gewählt aus einer Einfachbindung oder einer divalenten Gruppe abgeleitet von wahlweise mit Resten R² substituiertem Benzol, Biphenyl, Terphenyl, Fluoren, Spirobifluoren, Indenofluoren, Carbazol, Dibenzofuran, Dibenzothiophen, oder einer Kombination von zwei oder mehr dieser Gruppen, wobei nicht mehr als 30 aromatische Ringatome in der Gruppe E vorliegen;
A ist eine Gruppe der Formel (A-1) oder (A-3), bevorzugt eine Gruppe der Formel (A-1);
Ar² ist bei jedem Auftreten gleich oder verschieden gewählt aus einem aromatischen oder heteroaromatischen Ringsystem mit 6 bis 25 aromatischen Ringatomen, das mit einem oder mehreren Resten R² substituiert sein kann, und ist bevorzugt gewählt aus Phenyl, Biphenyl, Terphenyl, Fluorenyl, Spirobifluorenyl, Indenofluorenyl, Naphthyl, Phenanthrenyl, Furanyl, Benzofuranyl, Dibenzofuranyl, Thiophenyl, Benzothiophenyl, Dibenzothiophenyl, Carbazolyl, Indolocarbazolyl und Indenocarbazolyl, welche jeweils mit einem oder mehreren Resten R² substituiert sein können;
R¹ ist bei jedem Auftreten gleich oder verschieden H, D, F, CN, eine geradkettige Alkylgruppe mit 1 bis 10 C-Atomen oder eine verzweigte oder cyclische Alkylgruppe mit 3 bis 10 C-Atomen, oder ein aromatisches oder heteroaromatisches Ringsystem mit 6 bis 25 aromatischen Ringatomen, wobei die genannten Alkyl- und Alkoxygruppen und die genannten aromatischen oder heteroaromatischen Ringsysteme jeweils mit einem oder mehreren Resten R³ substituiert sein können, bevorzugt ist R¹ bei jedem Auftreten gleich oder verschieden H, F, CN, Methyl, tert-Butyl, Phenyl, Biphenyl, Dibenzofuran, Dibenzothiophen oder Carbazol, nochmals stärker bevorzugt gleich H.

Beispiele für Verbindungen gemäß der vorliegenden Erfindung sind:

| | | |
|---|---|---|
| 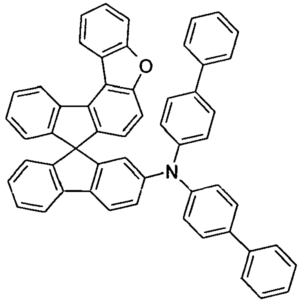 | 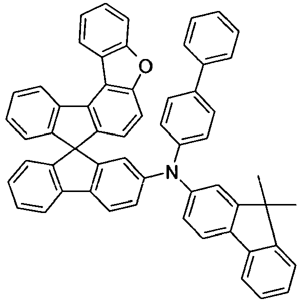 | 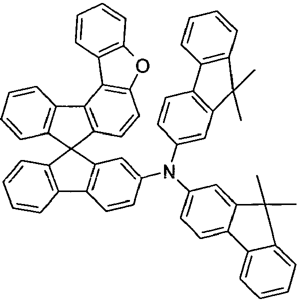 |
| 1 | 2 | 3 |
| 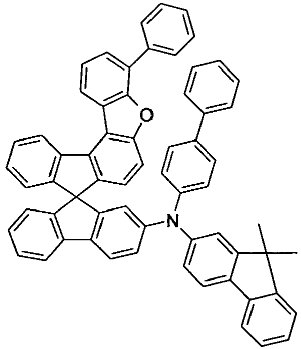 | 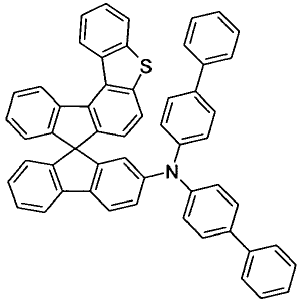 | 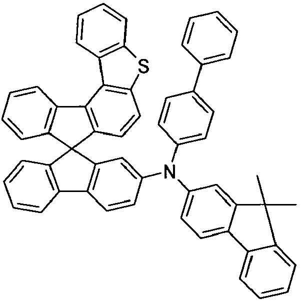 |
| 4 | 5 | 6 |
| 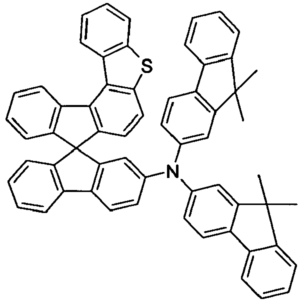 | 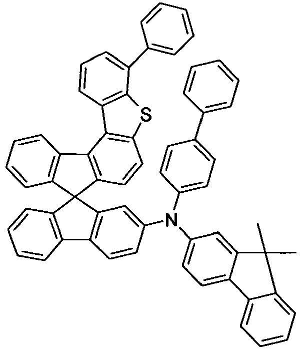 | 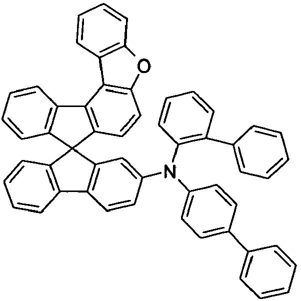 |
| 7 | 8 | 9 |
| 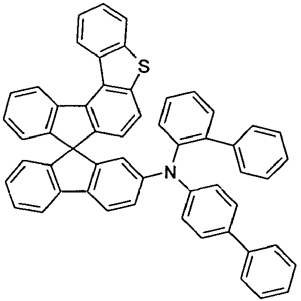 | 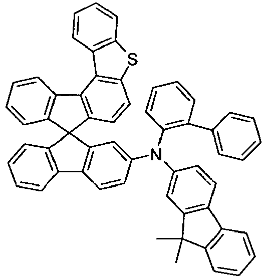 | 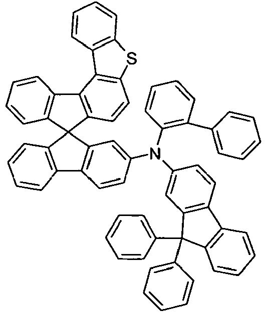 |
| 10 | 11 | 12 |
| 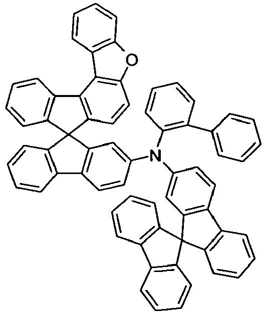 | 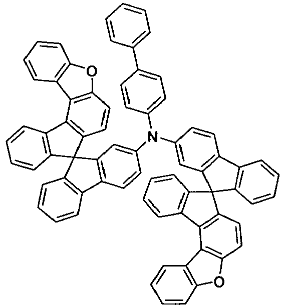 | 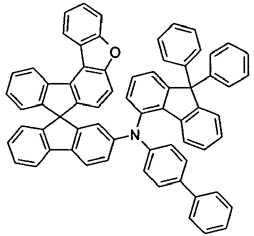 |
| 13 | 14 | 15 |
| 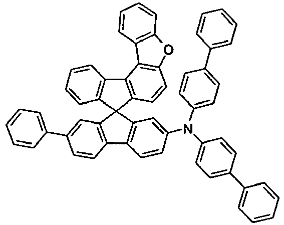 | 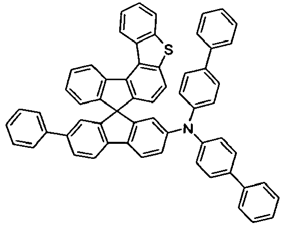 | 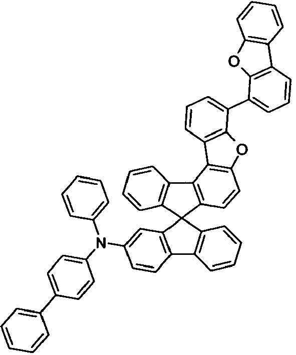 |
| 16 | 17 | 18 |
| 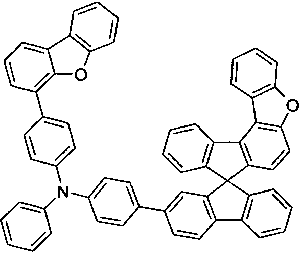 | 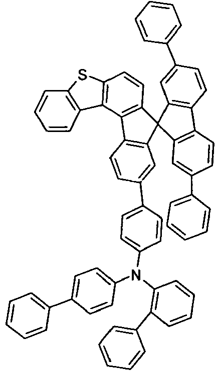 | 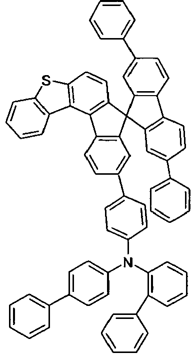 |
| 19 | 20 | 21 |
| 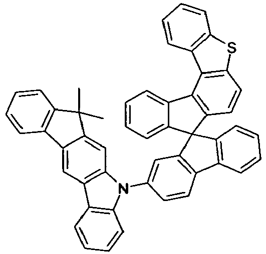 | 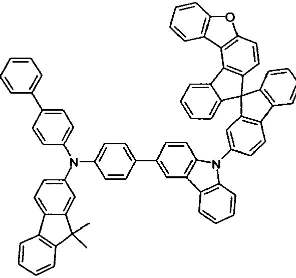 | 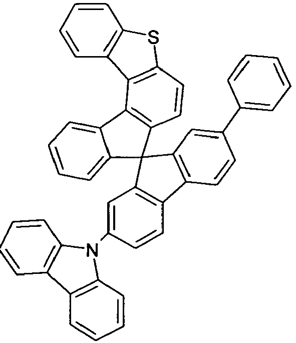 |
| 22 | 23 | 24 |
| 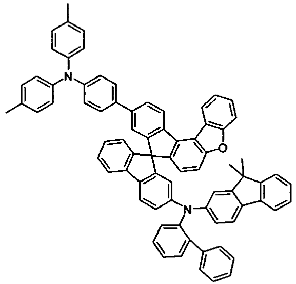 | 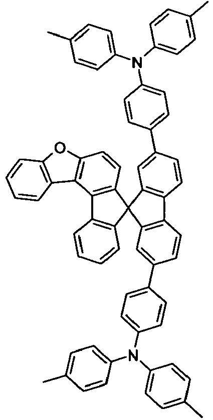 | 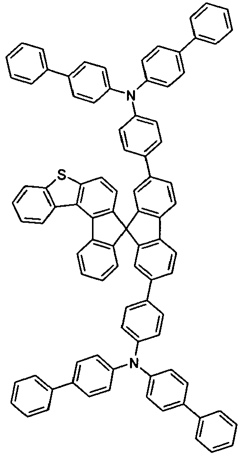 |
| 25 | 26 | 27 |
| 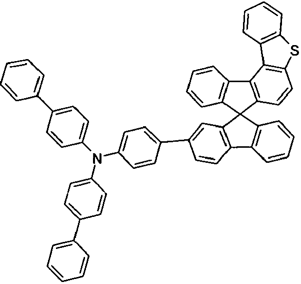 | 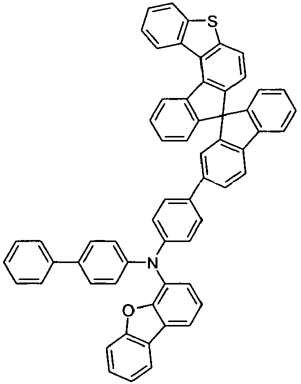 | 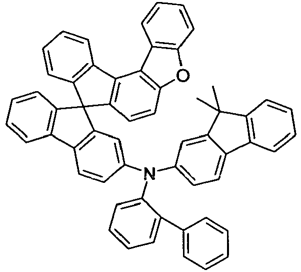 |
| 28 | 29 | 30 |
| 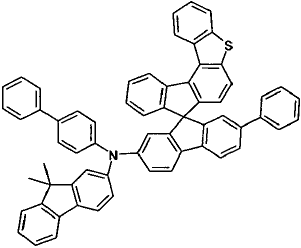 | 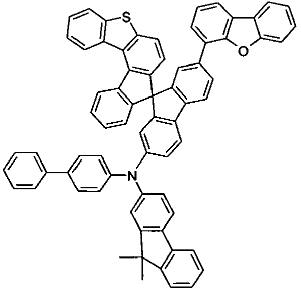 | 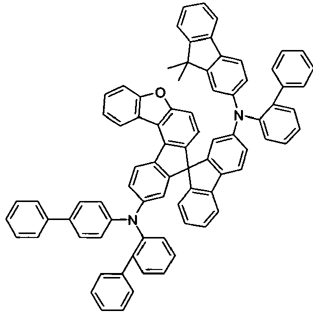 > |
| 31 | 32 | 33 |
| 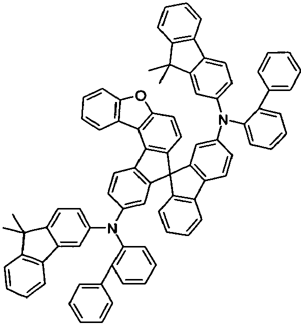 | 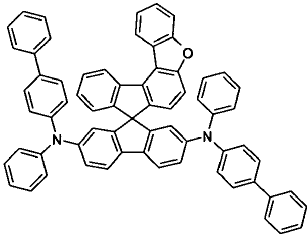 | 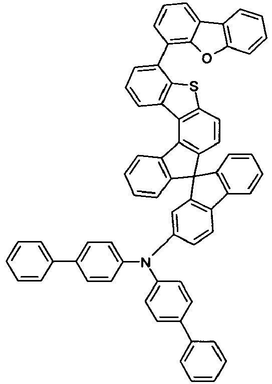 |
| 34 | 35 | 36 |
| 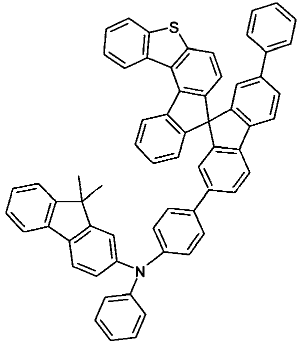 | 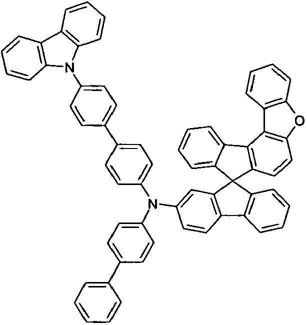 | 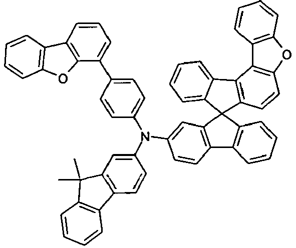 |
| 37 | 38 | 39 |
| 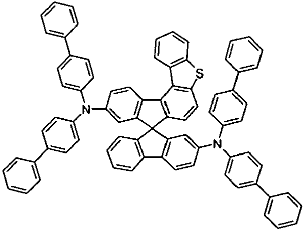 | 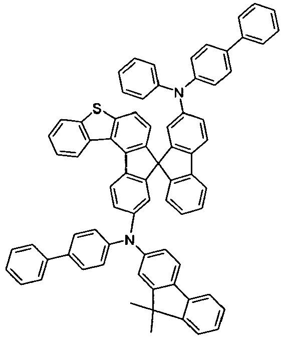 | 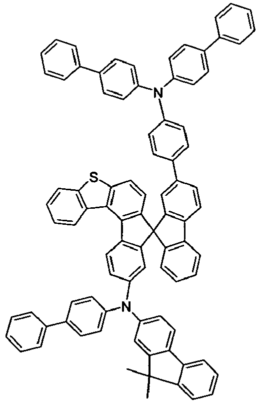 |
| 40 | 41 | 42 |
| 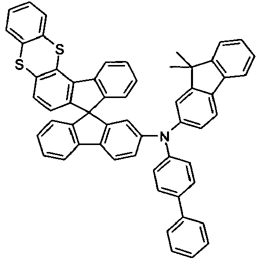 | 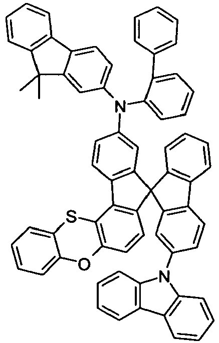 | 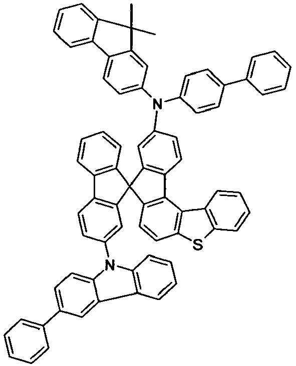 |
| 43 | 44 | 45 |
| 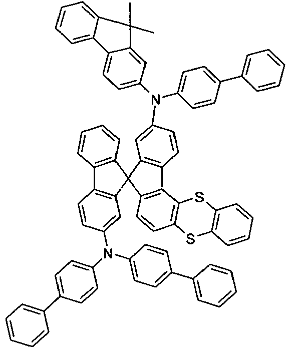 | 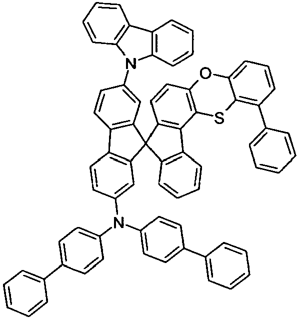 | 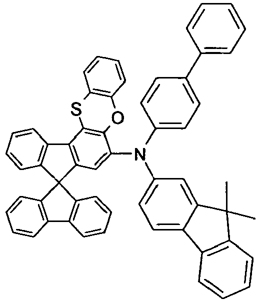 |
| 46 | 47 | 48 |
| 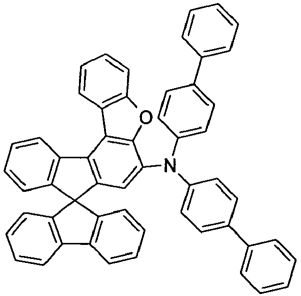 | 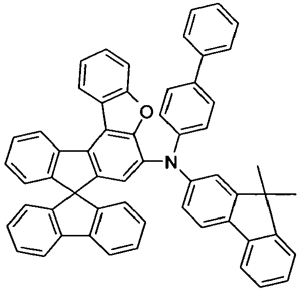 | 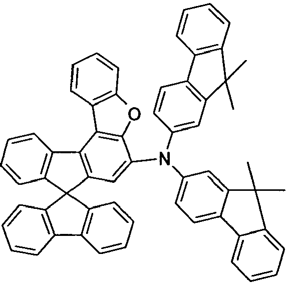 |
| 49 | 50 | 51 |
| 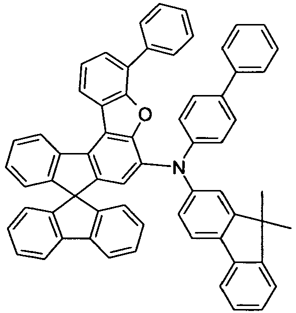 | 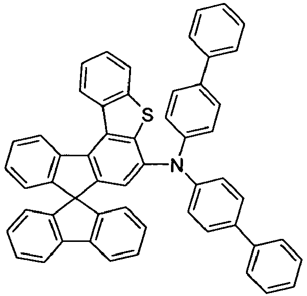 | 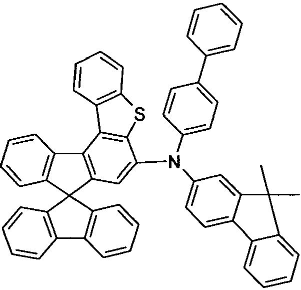 |
| 52 | 53 | 54 |
| 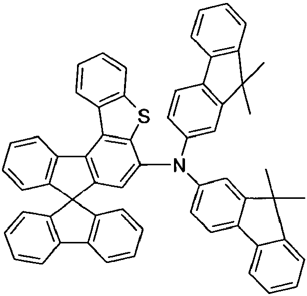 | 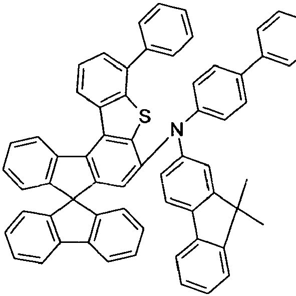 | 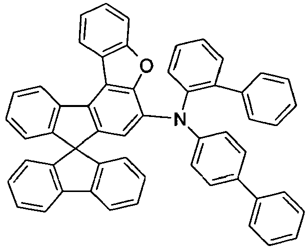 |
| 55 | 56 | 57 |
| 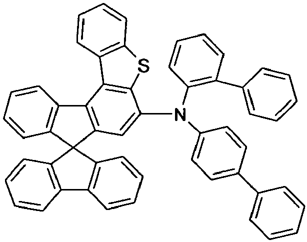 | 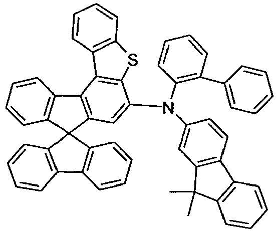 | 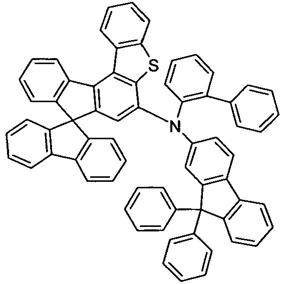 |
| 58 | 59 | 60 |
| 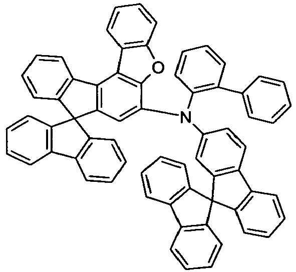 | 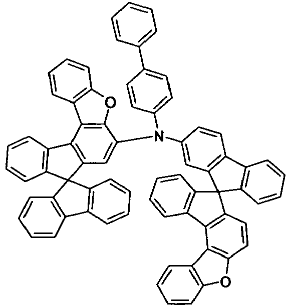 | 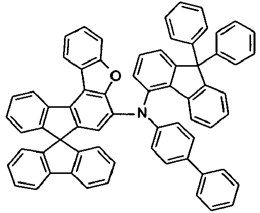 |
| 61 | 62 | 63 |
| 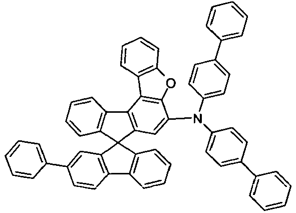 | 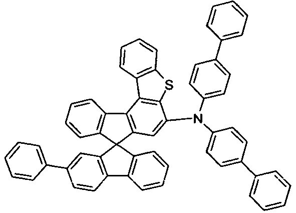 | 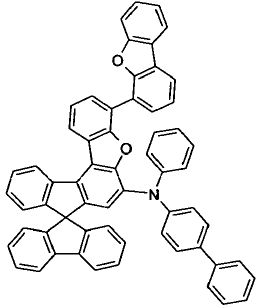 |
| 64 | 65 | 66 |
| 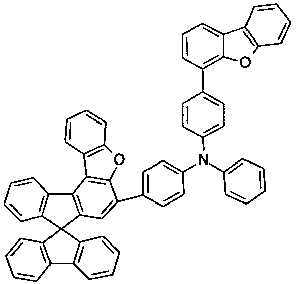 | 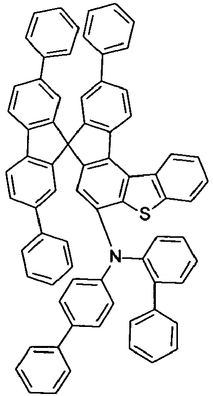 | 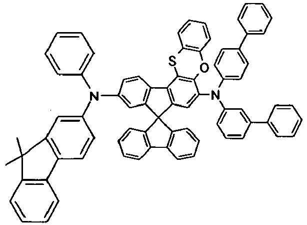 |
| 67 | 68 | 69 |
| 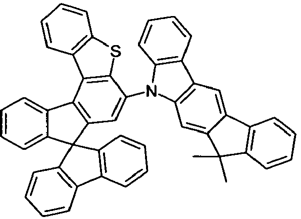 | 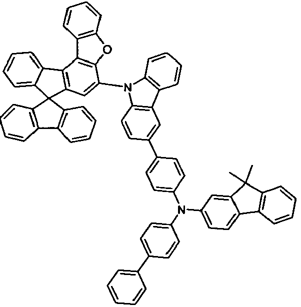 | 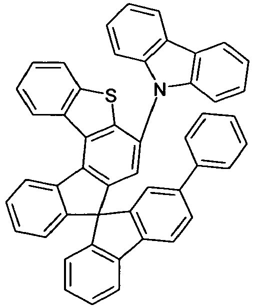 |
| 70 | 71 | 72 |
| 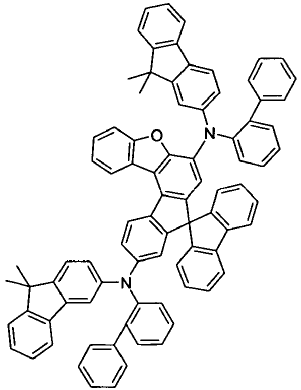 | 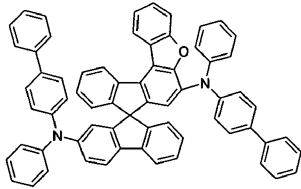 | 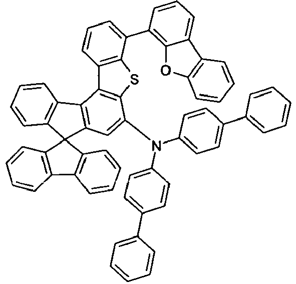 |
| 73 | 74 | 75 |
| 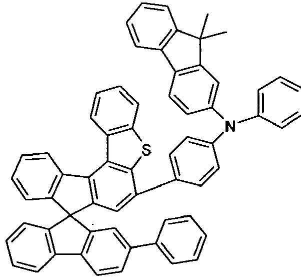 | 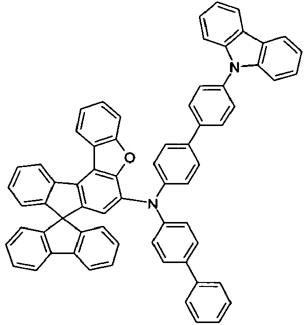 | 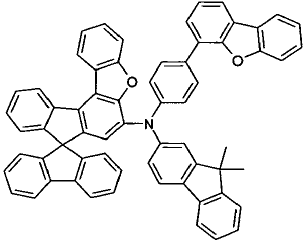 |
| 76 | 77 | 78 |
| 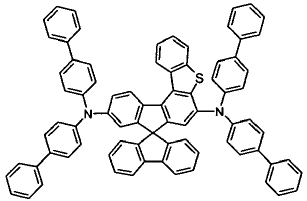 | 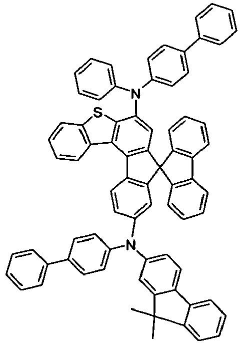 | 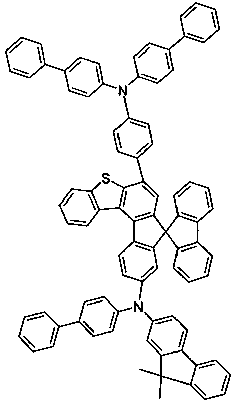 |
| 79 | 80 | 81 |
| 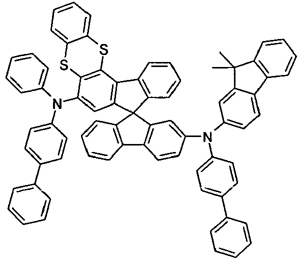 | 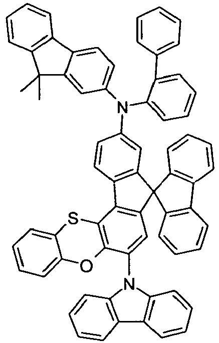 | 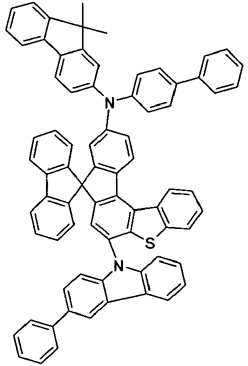 |
| 82 | 83 | 84 |

Die Synthese der Verbindungen gemäß Formel (I) kann unter Einsatz von Verfahren und Reaktionstypen durchgeführt werden, die im Stand der Technik bekannt sind, beispielsweise Halogenierung, metallorganische Addition, Buchwald-Kupplung und Suzuki-Kupplung.

Schemata 1 bis 3 zeigen mögliche Synthesewege zur Herstellung der erfindungsgemäßen Verbindungen. Sie dienen zur Erläuterung der Erfindung für den Fachmann, und sind nicht einschränkend auszulegen. Der Fachmann kann im Rahmen seines allgemeinen Fachwissens die gezeigten Synthesewege abwandeln, oder andere Wege entwickeln, falls dies vorteilhafter erscheint.

In den folgenden Syntheseschemata sind die Verbindungen unsubstituiert gezeigt. Dies schließt das Vorhandensein von beliebigen Substituenten in den Verfahren nicht aus.

In Schema 1 ist eine geeignete Synthese für die Zwischenstufe gemäß Formel (Z-I) gezeigt.

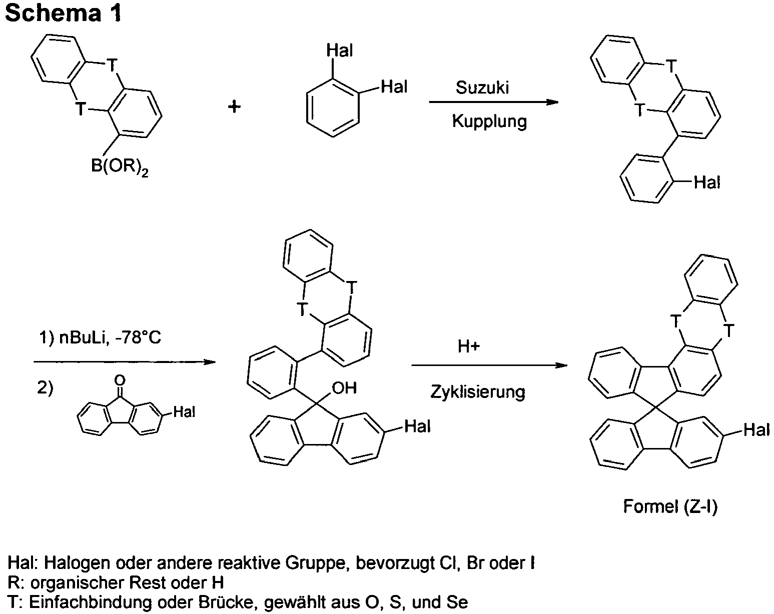

Analog dazu können die Zwischenstufen gemäß Formel (Z-II) hergestellt werden (Schema 2).

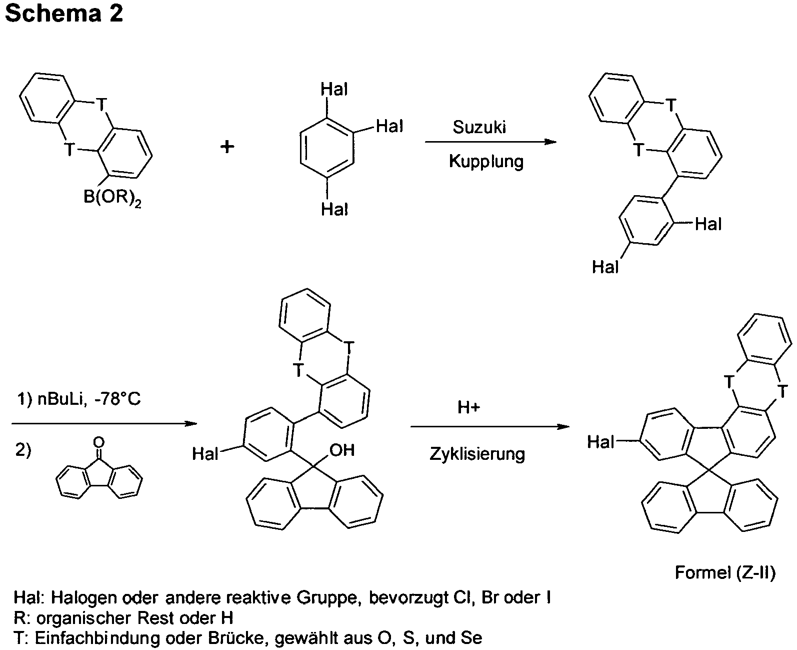

Über eine Abwandlung von Schema 1 bzw. 2 können Zwischenstufen hergestellt werden, die zwei oder mehr reaktive Gruppen Hal tragen. Hierzu werden sowohl ein Hal-substituierter Fluorenon-Baustein als auch eine mit drei Gruppen Hal substituierte Phenylgruppe eingesetzt. Alternativ kann auch ein zweifach oder mehrfach mit Hal substituierter Fluorenon-Baustein eingesetzt werden.

Die mit reaktiven Gruppen Z versehenen Zwischenstufen gemäß den Formeln (Z-I) bzw. (Z-II) sind vielseitige Bausteine, die zu Verbindungen der Formel (I) umgesetzt werden können, wie das folgende Schema zeigt:

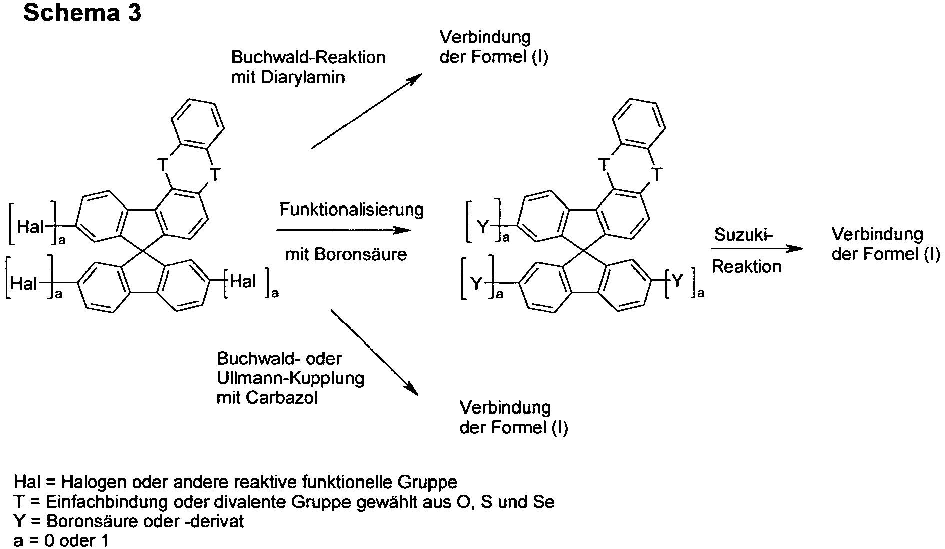

Gegenstand der vorliegenden Anmeldung ist daher auch ein Verfahren zur Herstellung von Verbindungen gemäß Formel (I), dadurch gekennzeichnet, dass zunächst das Spirobifluoren-Grundgerüst hergestellt wird, und in einem späteren Schritt über eine metallorganische Kupplungsreaktion eine Arylamino- oder eine Carbazolgruppe oder eine Aryl- oder Heteroarylgruppe, die mit einer Arylamino- oder Carbazolgruppe substituiert ist, eingeführt wird.

Die metallorganische Kupplungsreaktion ist dabei bevorzugt eine Buchwald-Kupplung oder eine Suzuki-Kupplung.

Die oben beschriebenen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere, enthaltend eine oder mehrere Verbindungen gemäß Formel (I), wobei die Bindung(en) zum Polymer, Oligomer oder Dendrimer an beliebigen, in Formel (I) mit R¹ oder R² substituierten Positionen lokalisiert sein können. Je nach Verknüpfung der Verbindung gemäß Formel (I) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (I) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formel (I) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (I) in Oligomeren, Dendrimeren und Polymeren gelten dieselben Bevorzugungen wie oben für Verbindungen gemäß Formel (I) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (I) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind die SUZUKI-Polymerisation, die YAMAMOTO-Polymerisation; die STILLE-Polymerisation; und die HARTWIG-BUCHWALD-Polymerisation.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (I) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (OLEDs). Abhängig von der Substitution werden die Verbindungen in unterschiedlichen Funktionen und Schichten eingesetzt.

Weiterer Gegenstand der Erfindung ist daher die Verwendung der Verbindung gemäß Formel (I) in einer elektronischen Vorrichtung. Dabei ist die elektronische Vorrichtung bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs).

Weiterer Gegenstand der Erfindung ist, wie bereits oben ausgeführt, eine elektronische Vorrichtung, enthaltend mindestens eine Verbindung gemäß Formel (I). Dabei ist die elektronische Vorrichtung bevorzugt ausgewählt aus den oben genannten Vorrichtungen.

Besonders bevorzugt ist sie eine organische Elektrolumineszenzvorrichtung (OLED), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (I) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen.

Die Abfolge der Schichten der organischen Elektrolumineszenzvorrichtung enthaltend die Verbindung der Formel (I) ist bevorzugt die folgende: Anode-Lochinjektionsschicht-Lochtransportschicht-wahlweise weitere Lochtransportschicht-wahlweise Elektronenblockierschicht-emittierende Schicht-wahlweise Lochblockierschicht-Elektronentransportschicht-Elektroneninjektionsschicht-Kathode. Es können zusätzlich weitere Schichten in der OLED vorhanden sein.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, grünes, gelbes, orangefarbenes oder rotes Licht emittieren. Insbesondere bevorzugt sind Dreischichtsysteme, also Systeme mit drei emittierenden Schichten, wobei die drei Schichten blaue, grüne und orangefarbene oder rote Emission zeigen (für den prinzipiellen Aufbau siehe z. B. WO 2005/011013). Die erfindungsgemäßen Verbindungen sind dabei bevorzugt in der Lochtransportschicht, Lochinjektionsschicht oder der Elektronenblockierschicht vorhanden.

Es ist erfindungsgemäß bevorzugt, wenn die Verbindung gemäß Formel (I) in einer elektronischen Vorrichtung enthaltend einen oder mehrere phosphoreszierende emittierende Verbindungen eingesetzt wird. Dabei kann die Verbindung in unterschiedlichen Schichten, bevorzugt in einer Lochtransportschicht, einer Elektronenblockierschicht, einer Lochinjektionsschicht oder in einer emittierenden Schicht, enthalten sein.

Vom Begriff phosphoreszierende emittierende Verbindungen sind typischerweise Verbindungen umfasst, bei denen die Lichtemission durch einen spin-verbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende emittierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende emittierende Verbindungen Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten. Dabei werden im Sinne der vorliegenden Erfindung alle lumineszierenden Iridium-, Platin- oder Kupferkomplexe als phosphoreszierende emittierende Verbindungen angesehen.

Beispiele für die oben beschriebenen emittierenden Verbindungen können den Anmeldungen WO 00/70655, WO 01/41512, WO 02/02714, WO 02/15645, EP 1191613, EP 1191612, EP 1191614, WO 05/033244, WO 05/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (I) in organischen Elektrolumineszenzvorrichtungen einsetzen. Weitere Beispiele sind in einer folgenden Tabelle aufgeführt.

Die Verbindung gemäß Formel (I) kann aber auch erfindungsgemäß in einer elektronischen Vorrichtung enthaltend eine oder mehrere fluoreszierende emittierende Verbindungen eingesetzt werden.

In einer bevorzugten Ausführungsform der Erfindung werden die Verbindungen gemäß Formel (I) als Lochtransportmaterial eingesetzt. Die Verbindungen liegen dann bevorzugt in einer Lochtransportschicht, einer Elektronenblockierschicht oder einer Lochinjektionsschicht vor. Besonders bevorzugt ist die Verwendung in einer Elektronenblockierschicht.

Eine Lochtransportschicht gemäß der vorliegenden Anmeldung ist eine Schicht mit lochtransportierender Funktion, welche sich zwischen Anode und emittierender Schicht befindet.

Lochinjektionsschichten und Elektronenblockierschichten werden im Sinne der vorliegenden Anmeldung als spezielle Ausführungsformen von Lochtransportschichten verstanden. Eine Lochinjektionsschicht ist dabei im Fall von mehreren Lochtransportschichten zwischen Anode und emittierender Schicht eine Lochtransportschicht, welche sich direkt an die Anode anschließt oder nur durch eine einzelne Beschichtung der Anode von ihr getrennt ist. Eine Elektronenblockierschicht ist im Fall von mehreren Lochtransportschichten zwischen Anode und emittierender Schicht diejenige Lochtransportschicht, welche sich direkt anodenseitig an die emittierende Schicht anschließt.

Wird die Verbindung gemäß Formel (I) als Lochtransportmaterial in einer Lochtransportschicht, einer Lochinjektionsschicht oder einer Elektronenblockierschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen eingesetzt werden. Gemäß einer bevorzugten Ausführungsform enthält die organische Schicht enthaltend die Verbindung der Formel (I) dann zusätzlich einen oder mehrere p-Dotanden. Als p-Dotanden werden gemäß der vorliegenden Erfindung bevorzugt solche organischen Elektronenakzeptorverbindungen eingesetzt, die eine oder mehrere der anderen Verbindungen der Mischung oxidieren können.

Besonders bevorzugte Ausführungsformen von p-Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600, WO 2012/095143 und DE 102012209523 offenbarten Verbindungen.

Besonders bevorzugt als p-Dotanden sind Chinodimethanverbindungen, Azaindenofluorendione, Azaphenalene, Azatriphenylene, I₂, Metallhalogenide, bevorzugt Übergangsmetallhalogenide, Metalloxide, bevorzugt Metalloxide enthaltend mindestens ein Übergangsmetall oder ein Metall der 3. Hauptgruppe, und Übergangsmetallkomplexe, bevorzugt Komplexe von Cu, Co, Ni, Pd und Pt mit Liganden enthaltend mindestens ein Sauerstoffatom als Bindungsstelle. Bevorzugt sind weiterhin Übergangsmetalloxide als Dotanden, bevorzugt Oxide von Rhenium, Molybdän und Wolfram, besonders bevorzugt Re₂O₇, MoO₃, WO₃ und ReO₃.

Die p-Dotanden liegen bevorzugt weitgehend gleichmäßig verteilt in den p-dotierten Schichten vor. Dies kann beispielsweise durch Co-Verdampfung des p-Dotanden und der Lochtransportmaterial-Matrix erreicht werden.

Bevorzugt sind als p-Dotanden insbesondere die folgenden Verbindungen:

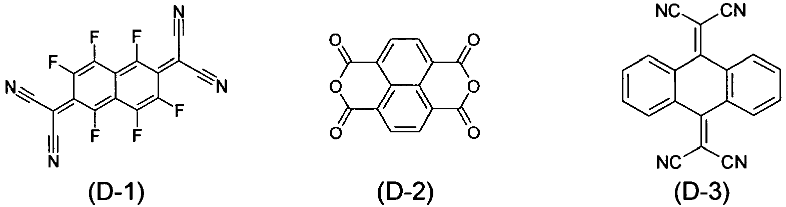

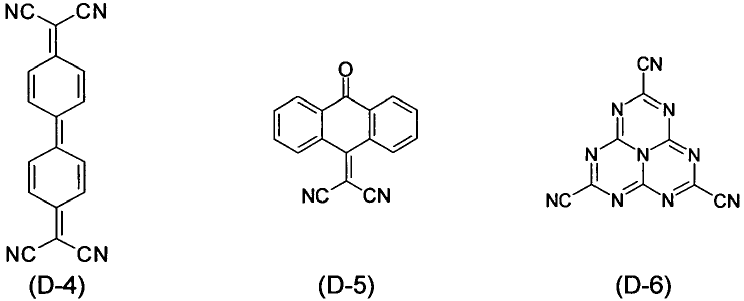

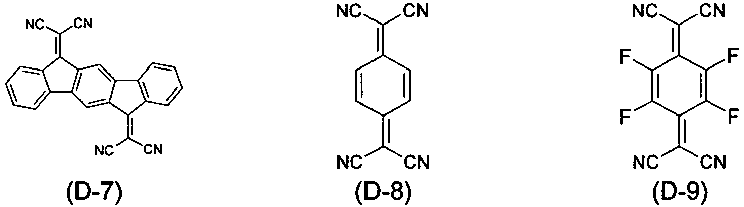

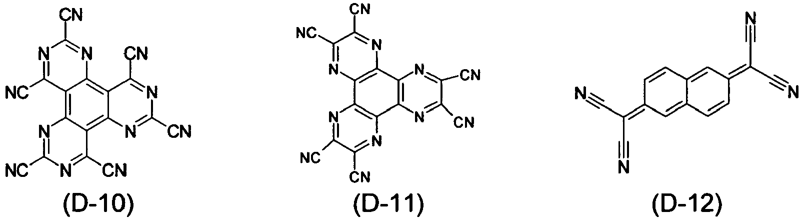

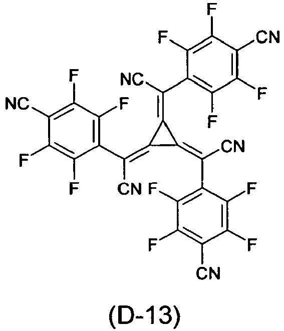

In einer weiteren bevorzugten Ausführungsform der Erfindung wird die Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

In einer weiteren Ausführungsform der vorliegenden Erfindung wird die Verbindung der Formel (I) in einer emittierenden Schicht als Matrixmaterial in Kombination mit einer oder mehreren emittierenden Verbindungen, vorzugsweise phosphoreszierenden emittierenden Verbindungen, eingesetzt.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil der emittierenden Verbindung zwischen 0.1 und 50.0 Vol.-%, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere emittierende Verbindungen enthalten. Auch in diesem Fall sind die emittierenden Verbindungen im Allgemeinen diejenigen Verbindungen, deren Anteil im System der kleinere ist und die Matrixmaterialien sind diejenigen Verbindungen, deren Anteil im System der größere ist. In Einzelfällen kann jedoch der Anteil eines einzelnen Matrixmaterials im System kleiner sein als der Anteil einer einzelnen emittierenden Verbindung.

Es ist bevorzugt, dass die Verbindungen gemäß Formel (I) als eine Komponente von Mixed-Matrix-Systemen verwendet werden. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die Verbindung der Formel (I) stellt dabei bevorzugt das Matrixmaterial mit lochtransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere emittierende Verbindungen umfassen, bevorzugt eine oder mehrere phosphoreszierende emittierende Verbindungen. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende emittierende Verbindungen oder den bevorzugten Matrixmaterialien für fluoreszierende emittierende Verbindungen, je nachdem welche Art von emittierender Verbindung im mixed-Matrix-System eingesetzt wird.

Bevorzugte phosphoreszierende emittierende Verbindungen zur Verwendung in Mixed-Matrix-Systemen sind dieselben, wie sie weiter oben als allgemein bevorzugte phosphoreszierende Emittermaterialien aufgeführt wurden.

Im Folgenden werden bevorzugte Ausführungsformen für die verschiedenen Funktionsmaterialien der elektronischen Vorrichtung aufgeführt.

Bevorzugte phosphoreszierende emittierende Verbindungen sind die oben genannten.

Bevorzugte fluoreszierende emittierende Verbindungen sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte emittierende Verbindungen sind Indenofluorenamine bzw. - diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen. Ebenfalls bevorzugt sind die in WO 2012/048780 und die in WO 2013/185871 offenbarten Pyren-Arylamine. Ebenfalls bevorzugt sind die in WO 2014/037077 offenbarten Benzoindenofluoren-Amine, die in WO 2014/106522 offenbarten Benzofluoren-Amine und die in WO 2014/111269 offenbarten erweiterten Benzoindenofluorene.

Als Matrixmaterialien, bevorzugt für fluoreszierende emittierende Verbindungen, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind. Bevorzugt sind weiterhin die in WO 2006/097208, WO 2006/131192, WO 2007/065550, WO 2007/110129, WO 2007/065678, WO 2008/145239, WO 2009/100925, WO 2011/054442, und EP 1553154 offenbarten Anthracenderivate, sowie die in EP 1749809, EP 1905754 und US 2012/0187826 offenbarten Pyren-Verbindungen.

Bevorzugte Matrixmaterialien für phosphoreszierende emittierende Verbindungen sind neben den Verbindungen der Formel (I) aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109, WO 2011/000455 oder WO 2013/041176, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol-bzw. Tetraazasilol-Derivate, z. B. gemäß WO 2010/054729, Diazaphosphol-Derivate, z. B. gemäß WO 2010/054730, überbrückte CarbazolDerivate, z. B. gemäß US 2009/0136779, WO 2010/050778, WO 2011/042107, WO 2011/088877 oder WO 2012/143080, Triphenylenderivaten, z. B. gemäß WO 2012/048781, oder Lactame, z. B. gemäß WO 2011/116865 oder WO 2011/137951.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind neben den Verbindungen der Formel (I) beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Bevorzugt umfasst die erfindungsgemäße OLED zwei oder mehr unterschiedliche lochtransportierende Schichten. Die Verbindung der Formel (I) kann dabei in einer oder in mehreren oder in allen lochtransportierenden Schichten eingesetzt werden. Gemäß einer bevorzugten Ausführungsform wird die Verbindung der Formel (I) in genau einer lochtransportierenden Schicht eingesetzt, und in den weiteren vorhandenen lochtransportierenden Schichten werden andere Verbindungen eingesetzt, bevorzugt aromatische Aminverbindungen.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise Alq₃, Zirkoniumkomplexe, beispielsweise Zrq₄, Lithiumkomplexe, beispielsweise Liq, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, AI, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder AI, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die elektronische Vorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine elektronische Vorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine elektronische Vorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) nötig. Hohe Löslichkeit lässt sich durch geeignete Substitution der Verbindungen erreichen.

Weiterhin bevorzugt ist es, dass zur Herstellung einer erfindungsgemäßen elektronischen Vorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß Formel (I) in Displays, als Lichtquellen in Beleuchtungsanwendungen sowie als Lichtquellen in medizinischen und/oder kosmetischen Anwendungen (z.B. Lichttherapie) eingesetzt werden.

### Ausführungsbeispiele

### A) Synthesebeispiele

### Beispiel 1: Synthese der Verbindungen (1-1) bis (1-26)

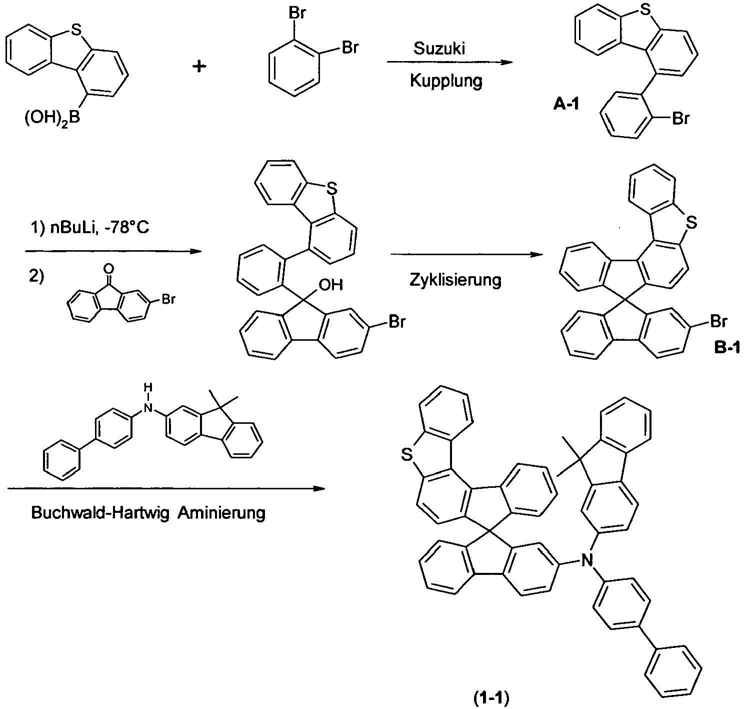

### Synthese von 1-(2-Bromo-phenyl)-dibenzothiophen A-1

80 g (351 mmol) Dibenzothiophen-1-Boronsäure (CAS: 1245943-60-5), 83 g (351 mmol) 1,2-Dibrombenzol und 8,2 g (7,02 mmol) Pd(Ph₃P)₄ werden in 700 mL Dioxan suspendiert. Zu dieser Suspension werden 440 mL (877 mmol) Kaliumcarbonat Lösung 2 M langsam addiert, und die Reaktionsmischung wird 18 h unter Rückfluss erhitzt. Nach Erkalten wird die organische Phase abgetrennt, über Kieselgel filtriert, dreimal mit 200 mL Wasser gewaschen und anschließend zur Trockne eingeengt. Der Rückstand wird durch Chromatographie an Kieselgel gereinigt. Ausbeute: 101 g (297 mmol), 85% d. Th., Reinheit nach HPLC >97%.

Analog zu der beschriebenen Synthese von Verbindung A-1 werden die folgenden Verbindungen hergestellt:

| | Edukt 1 | Edukt 2 | Produkt | Ausbeute |
|---|---|---|---|---|
| **A-2** | 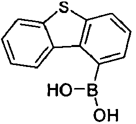 | 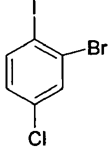 | 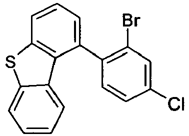 | 70% |
| | 1245943-60-5 | | | |
| **A-3** | 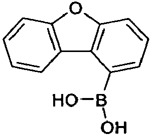 | 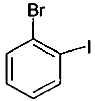 | 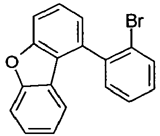 | 75% |
| | 162607-19-4 | | | |
| **A-4** | 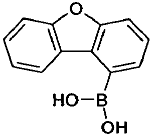 | 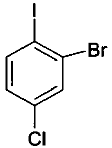 | 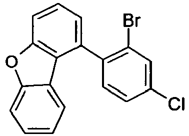 | 60% |
| | 162607-19-4 | | | |
| **A-5** | 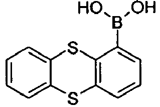 | 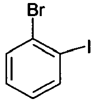 | 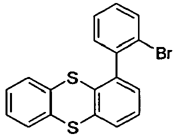 | 55% |
| | 108847-76-3 | | | |
| **A-6** | 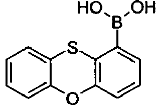 | 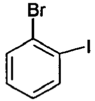 | 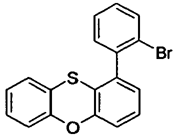 | 67% |
| | 636607-99-3 | | | |
| **A-13** | 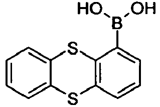 | 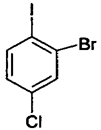 | 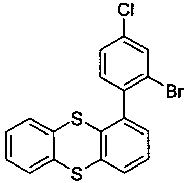 | 73% |
| | 108847-76-3 | | | |
| **A-14** | 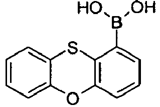 | 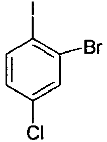 | 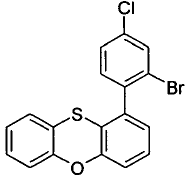 | 81% |
| | 636607-99-3 | | | |

### Synthese der Zwischenstufe B-1

56,3g (166 mmol) 1-(2-Bromo-phenyl)-dibenzothiophen A-1 werden in 700 mL THF bei -78°C vorgelegt. Bei dieser Temperatur werden 70 mL BuLi (2,5 M in Hexan) zugetropft. Nach 1 Stunde werden 45,2 g (174 mmol) 2-Brom-Fluoren-9-on in 200 mL THF zugetropft. Der Ansatz wird über Nacht bei Raumtemperatur rühren gelassen, auf Eiswasser gegeben und mit Dichlormethan extrahiert. Die vereinigten organischen Phasen werden mit Wasser gewaschen und über Natriumsulfat getrocknet. Das Lösungsmittel wird im Vakuum entfernt und der Rückstand wird ohne weitere Aufreinigung mit 90 mL HCl und 1L AcOH bei 75°C über Nacht erhitzt. Nach Erkalten saugt man vom ausgefallenen Feststoff ab, wäscht diesen zweimal mit 150 ml Wasser, dreimal mit je 150 ml Ethanol und kristallisiert abschließend aus Heptan um. Ausbeute: 59g (117 mmol), 71 %; Reinheit ca. 98 % nach ¹H-NMR.

Analog zu der beschriebenen Synthese von Verbindung B-1 werden die folgenden Verbindungen hergestellt:

| | Edukt 1 | Edukt 2 | Produkt | Ausbeute |
|---|---|---|---|---|
| **B-2** | 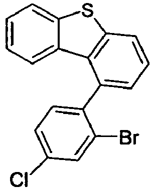 | 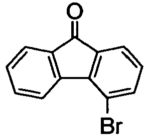 | 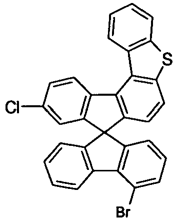 | 62% |
| | | 4269-17-4 | | |
| **B-3** | 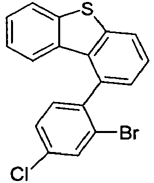 | 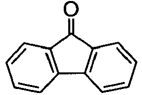 | 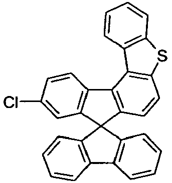 | 70% |
| | | 486-25-9 | | |
| **B-4** | 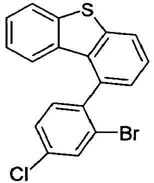 | 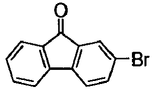 | 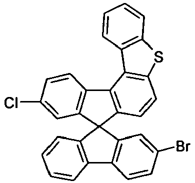 | 70% |
| | | 3096-56-8 | | |
| **B-5** | 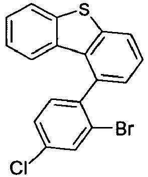 | 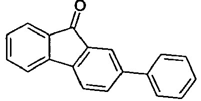 | 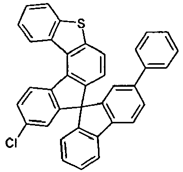 | 81% |
| | | 3096-49-9 | | |
| **B-6** | 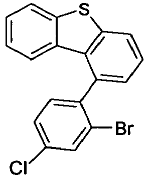 | 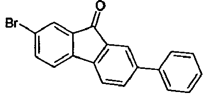 | 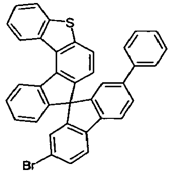 | 75% |
| | | 154851-21-3 | | |
| **B-7** | 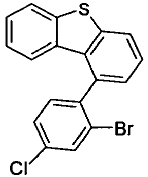 | 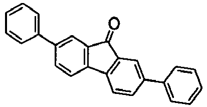 | 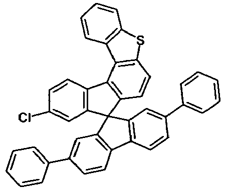 | 60% |
| | | 115033-91-5 | | |
| **B-8** | 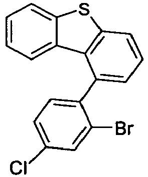 | 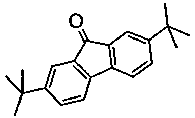 | 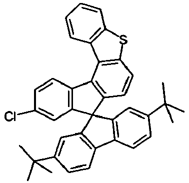 | 68% |
| | | 58775-13-6 | | |
| **B-9** | 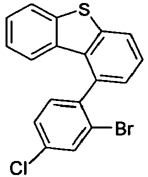 | 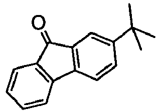 | 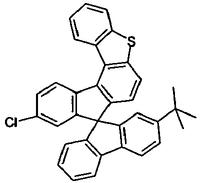 | 75% |
| | | 58775-11-4 | | |
| **B-10** | 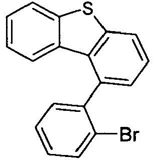 | 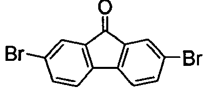 | 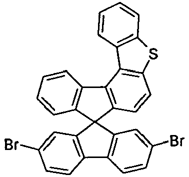 | 62% |
| | | 14348-75-5 | | |
| **B-11** | 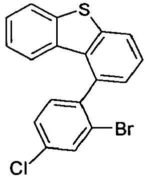 | 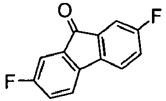 | 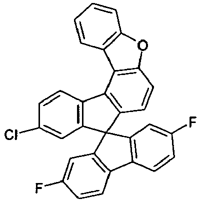 | 62% |
| | | 24313-53-9 | | |
| **B-11** | 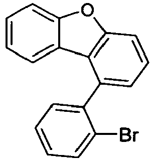 | 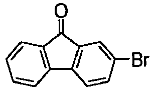 | 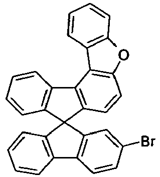 | 70% |
| | | 3096-56-8 | | |
| **B-12** | 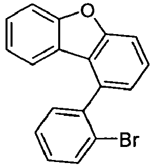 | 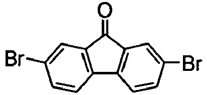 | 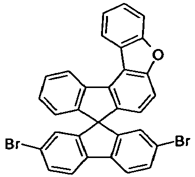 | 65% |
| | | 14348-75-5 | | |
| **B-13** | 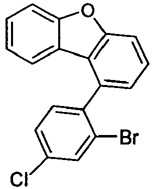 | 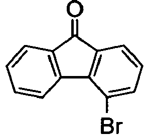 | 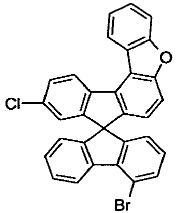 | 75% |
| | | 4269-17-4 | | |
| **B-14** | 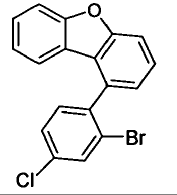 | 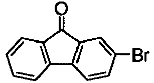 | 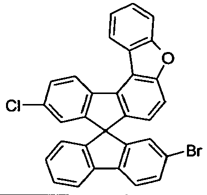 | 68% |
| | | 3096-56-8 | | |
| **B-17** | 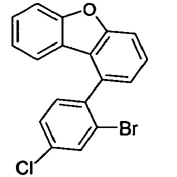 | 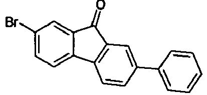 | 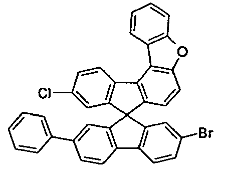 | 67% |
| | | 154851-21-3 | | |
| **B-18** | 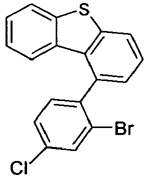 | 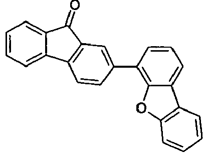 | 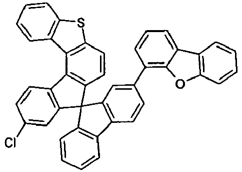 | 74% |
| **B-19** | 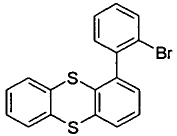 | 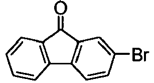 | 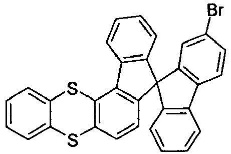 | 65% |
| | | 3096-56-8 | | |
| **B-20** | 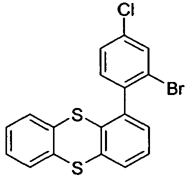 | 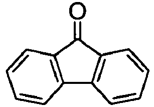 | 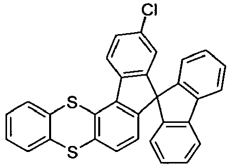 | 72% |
| | | 486-25-9 | | |
| **B-21** | 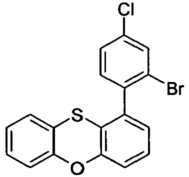 | 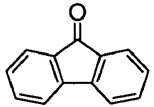 | 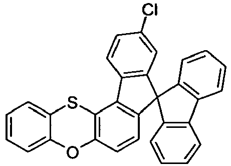 | 68% |
| | | 486-25-9 | | |

### Synthese der Verbindung (1-1)

13,7 g (38 mmol) Biphenyl-4-yl-(9,9-dimethyl-9H-fluoren-2-yl)-amin und 17,4 g (38 mol) des Bromo-Spiroderivats B-1 werden in 300 mL Toluol gelöst. Die Lösung wird entgast und mit N₂ gesättigt. Danach wird sie mit 1,52 mL (1,52 mmol) einer 1M Tri-tert-Butylphosphin Lösung und 170 mg (0,76 mmol) Pd(AcO)₂ versetzt und anschließend werden 9,0 g Natrium-tert-butylat (94,2 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9% (HPLC). Die Ausbeute von Verbindung (**1-1**) beträgt 22,7 g (77% der Theorie).

### Synthese der Verbindungen (1-2) bis (1-26)

Analog zu der in Beispiel 1 beschriebenen Synthese von Verbindung (**1-1**) werden auch die folgenden Verbindungen (**1-2**) bis (**1-26**) hergestellt.

| | Edukt 1 | Edukt 2 | Produkt | Ausbeute |
|---|---|---|---|---|
| **1-2** | 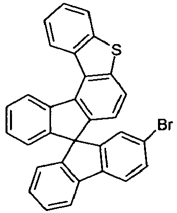 | 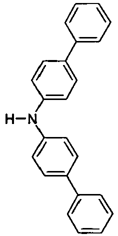 | 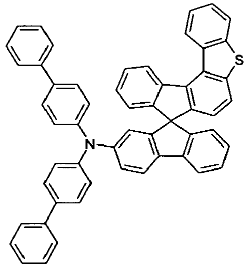 | 75 % |
| **1-3** | 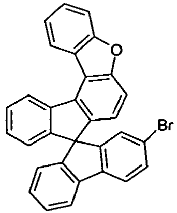 | 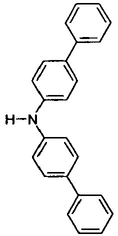 | 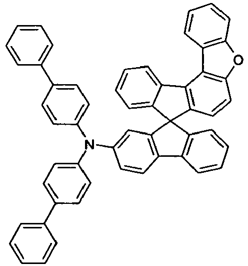 | 75 % |
| **1-4** | 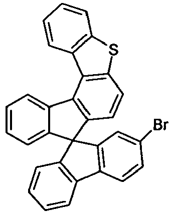 | 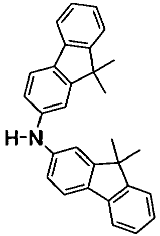 | 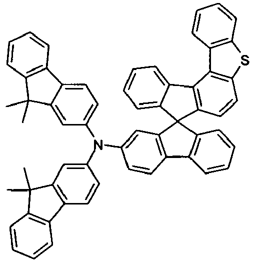 | 73% |
| **1-5** | 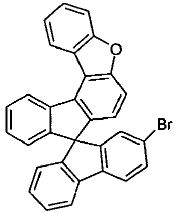 | 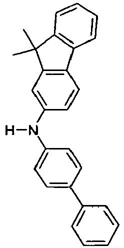 | 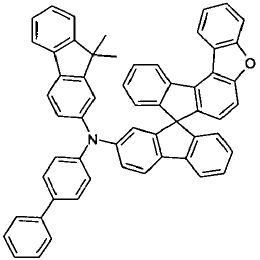 | 78% |
| **1-6** | 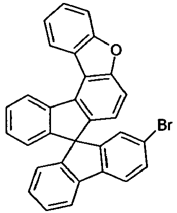 | 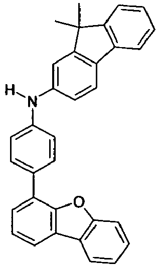 | 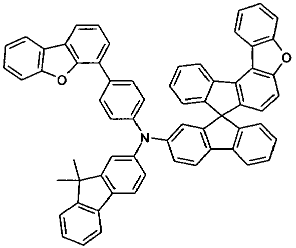 | 80% |
| **1-7** | 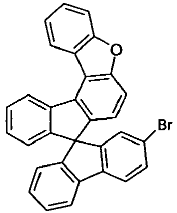 | 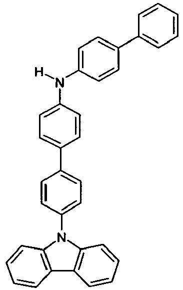 | 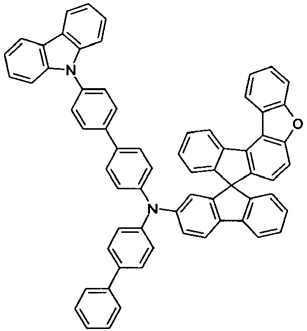 | 70% |
| **1-8** | 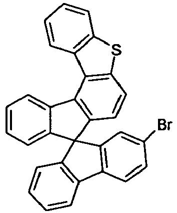 | 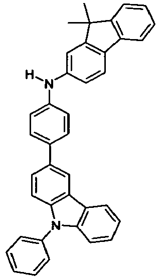 | 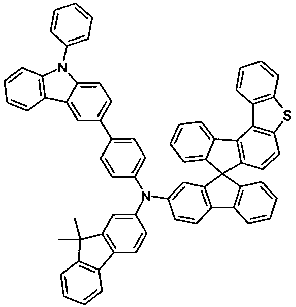 | 75% |
| **1-9** | 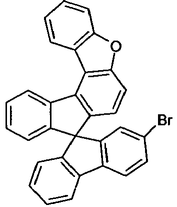 | 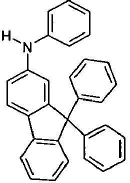 | 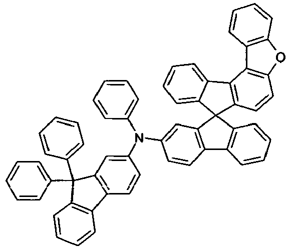 | 81% |
| **1-10** | 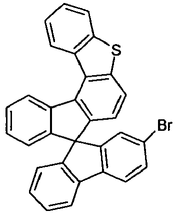 | 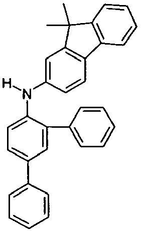 | 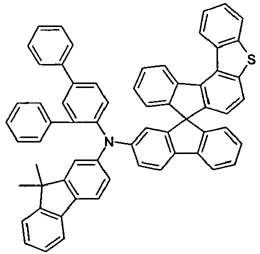 | 78% |
| **1-11** | 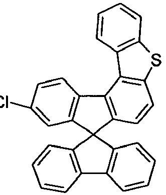 | 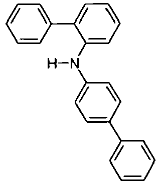 | 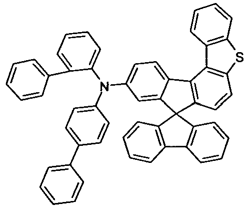 | 70% |
| **1-12** | 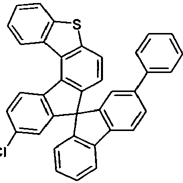 | 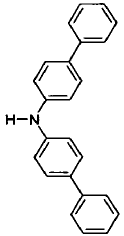 | 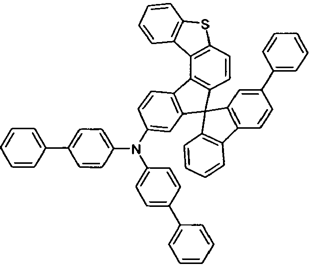 | 76% |
| **1-13** | 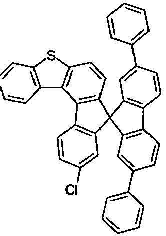 | 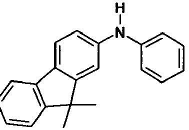 | 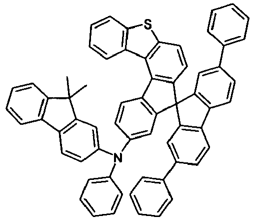 | 70% |
| **1-14** | 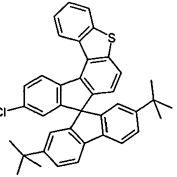 | 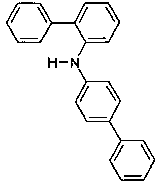 | 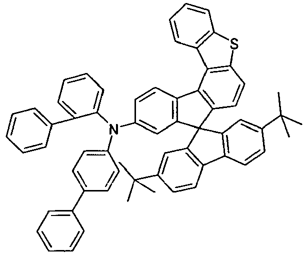 | 65% |
| **1-15** | 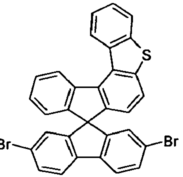 | 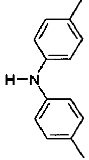 | 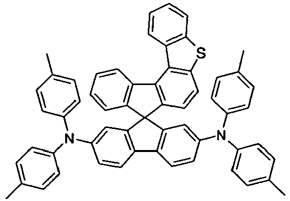 | 60% |
| | | (2 Äq) | | |
| **1-16** | 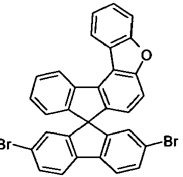 | 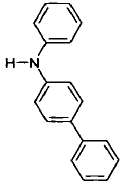 | 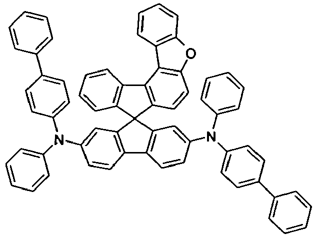 | 70% |
| | | (2 Äq) | | |
| **1-17** | 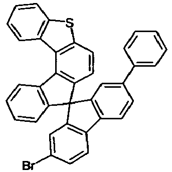 | 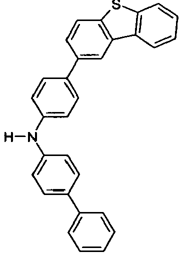 | 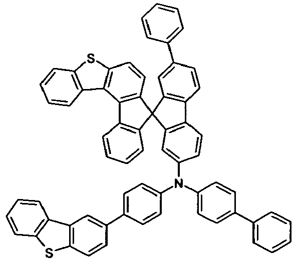 | 78% |
| **1-18** | 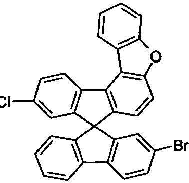 | 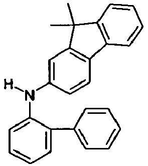 | 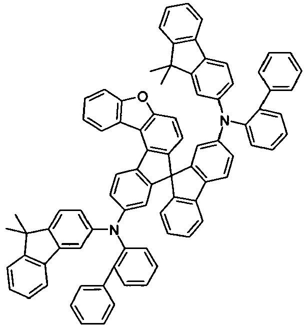 | 80% |
| | | (2 Äq) | | |
| **1-19** | 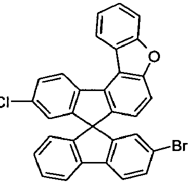 | 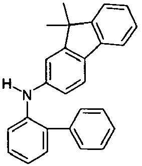 | 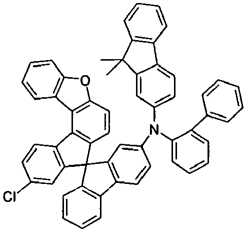 | 67% |
| **1-20** | 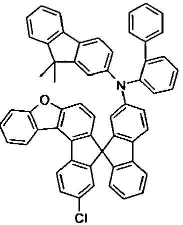 | 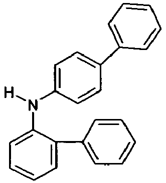 | 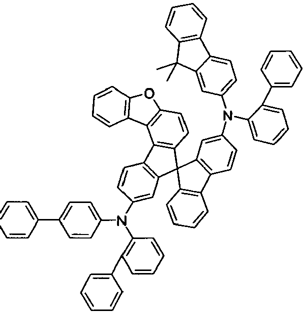 | 75% |
| **1-21** | 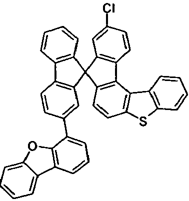 | 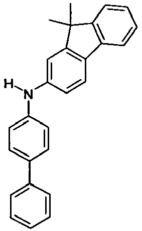 | 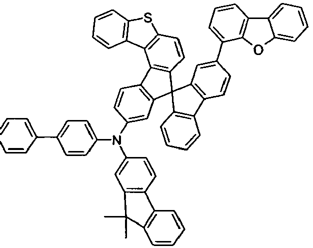 | 80% |
| **1-22** | 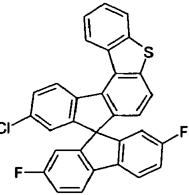 | 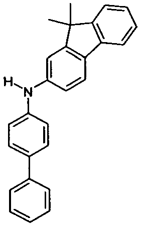 | 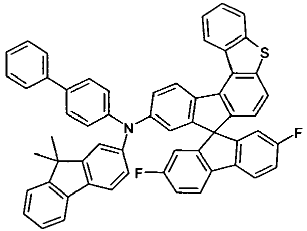 | 75% |
| **1-23** | 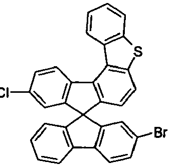 | 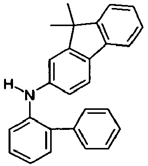 | 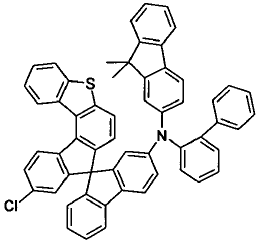 | 80% |
| **1-24** | 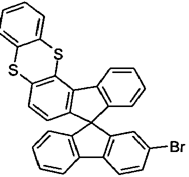 | 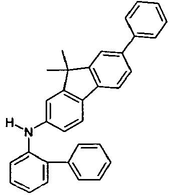 | 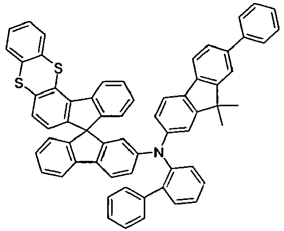 | 65% |
| **1-25** | 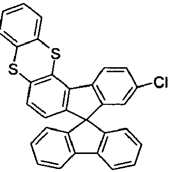 | 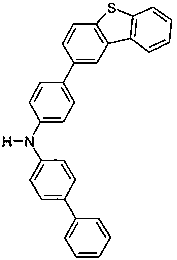 | 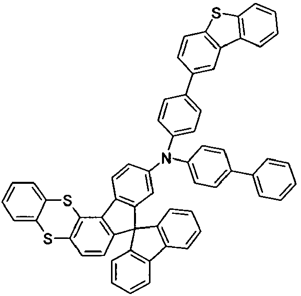 | 77% |
| **1-26** | 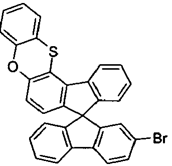 | 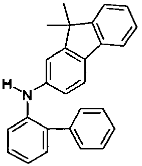 | 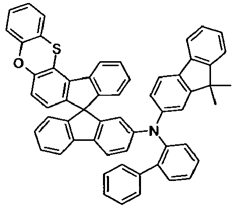 | 67% |

### Beispiel 2: Synthese der Verbindungen 2-1 bis 2-9

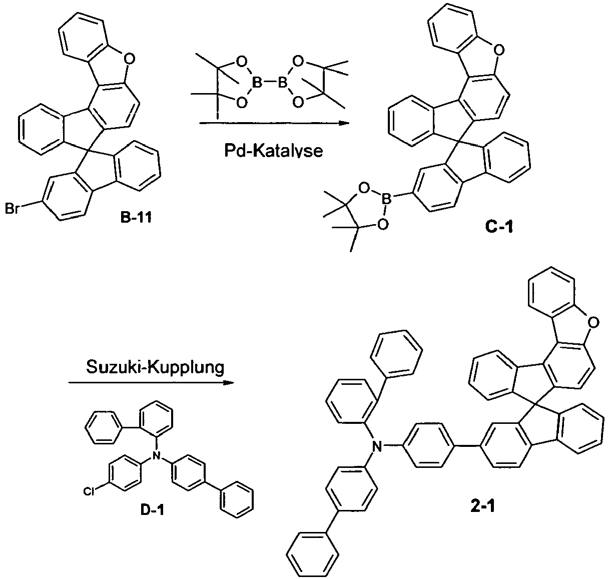

### Spirofluoren-boronester-Derivat (C-1)

36 g (74,2 mmol) des Spirofluoren-bromderivats B11, 22,6 g (89 mmol) Bis(pinacolato)diboran und 21,8 g (222 mmol) Kaliumacetat werden in 400 ml DMF suspendiert. Zu dieser Suspension werden 1,82 g (2,23 mmol) 1,1-Bis(diphenylphosphino)ferrocen-dichloropalladium(II) Komplex mit DCM gegeben. Die Reaktionsmischung wird 16 h unter Rückfluss erhitzt. Nach Erkalten wird die organische Phase abgetrennt, dreimal mit 400 mL Wasser gewaschen und anschließend zur Trockne eingeengt. Der Rückstand wird aus Toluol umkristallisiert (37 g, 94% Ausbeute).

Analog dazu werden folgende Verbindungen hergestellt:

| | Edukt | Produkt | Ausbeute |
|---|---|---|---|
| **C-2** | 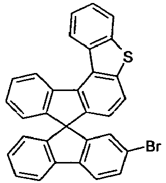 | 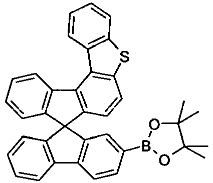 | 62% |
| **C-3** | 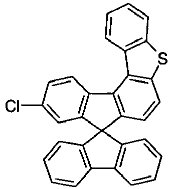 | 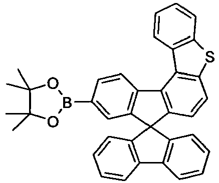 | 70% |
| **C-4** | 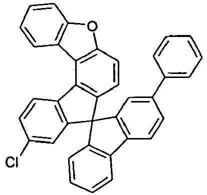 | 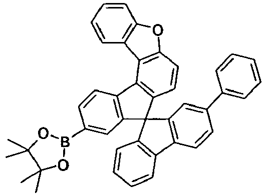 | 70% |
| **C-5** | 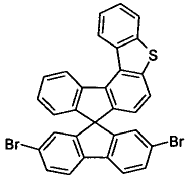 | 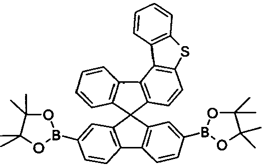 | 81% |
| **C-6** | 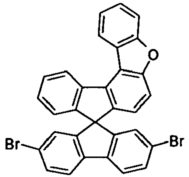 | 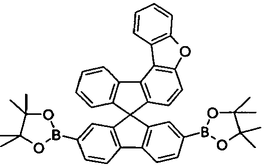 | 87% |
| **C-7** | 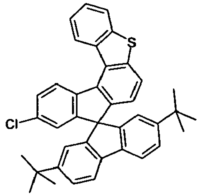 | 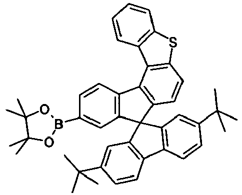 | 85% |
| **C-10** | 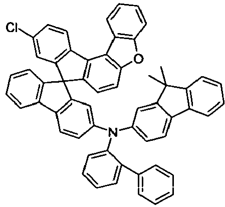 | 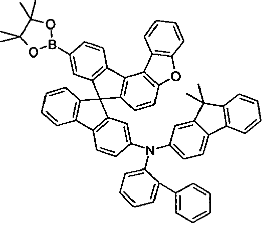 | 70% |

### Biphenyl-2-yl-biphenyl-4-yl-(4-chloro-phenyl)-amin (D-1)

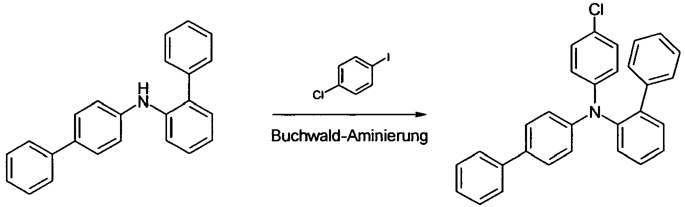

23,8 g Biphenyl-2-yl-biphenyl-4-yl-amin (74 mmol) und 21,2 g 4-Chloriodbenzol (89 mmol) werden in 500 mL Toluol gelöst. Die Lösung wird entgast und mit N₂ gesättigt. Danach wird sie mit 3 mL (3 mmol) einer Tri-tert-Butylphosphin 1M-Lösung und 0,33 g (1,48 mmol) Palladium(II)acetat versetzt und anschließend werden 10,7 g Natrium-tert-butylat (111 mmol) zugegeben. Die Reaktionsmischung wird 12 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert. Die Ausbeute beträgt 29 g (90% d. Th).

Analog dazu werden folgende Verbindungen hergestellt:

| | Edukt 1 | Edukt 2 | Produkt | Ausbeute |
|---|---|---|---|---|
| **D-2** | 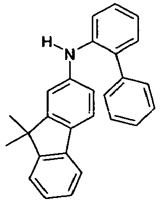 | 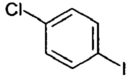 | 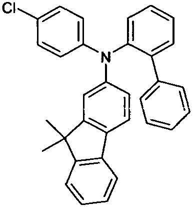 | 78 % |
| **D-3** | 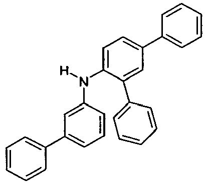 | 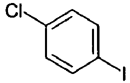 | 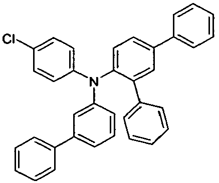 | 80% |
| **D-4** | 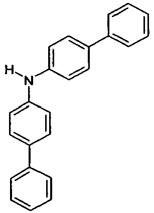 | 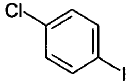 | 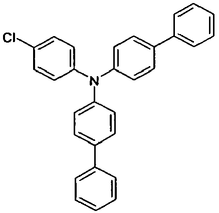 | 81% |
| **D-5** | 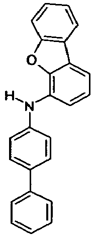 | 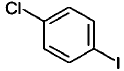 | 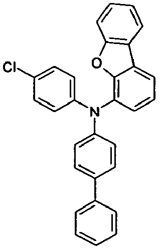 | 92% |
| **D-6** | 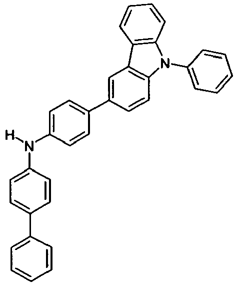 | 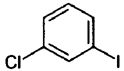 | 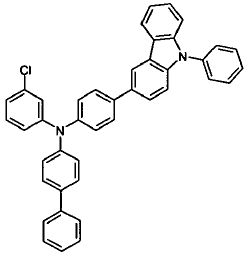 | 85% |
| **D-7** | 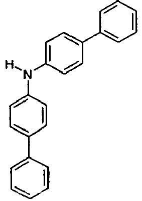 | 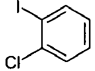 | 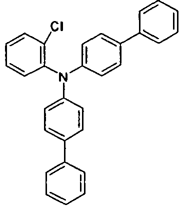 | 75% |
| **D-8** | 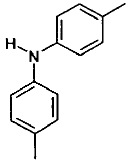 | 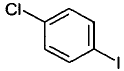 | 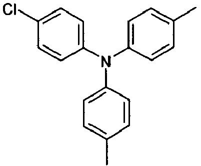 | 89% |

### Synthese der Verbindung (2-1)

18,0 g (32 mmol) Spirofluoren-pinacolboronester Derivat C-1 und 15,3 g (32 mmol) Chlor-Derivat D-1 werden in 360 mL Dioxan und 9,8 g Caesiumfluorid (64 mmol) suspendiert. Zu dieser Suspension werden 1,19 g (1,6 mmol) Palladium dichlorid-bis(tricyclohexylphosphin) gegeben, und die Reaktionsmischung wird 16 h unter Rückfluss erhitzt. Nach Erkalten wird die organische Phase abgetrennt, über Kieselgel filtriert, dreimal mit 100 mL Wasser gewaschen und anschließend zur Trockne eingeengt. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 18 g (70% d. Th).

### Synthese der Verbindungen (2-2) bis (2-9)

Analog zu der beschriebenen Synthese von Verbindung (**2-1**) werden auch die folgenden Verbindungen (**2-2**) bis (**2-9**) hergestellt.

| | Edukt 1 | Edukt 2 | Produkt | Ausbeute |
|---|---|---|---|---|
| **2-2** | 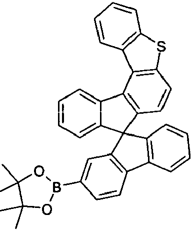 | 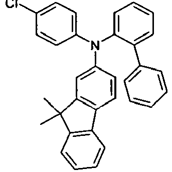 | 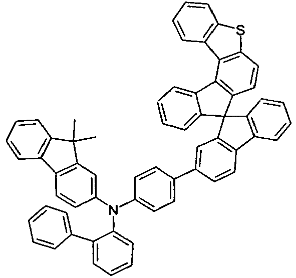 | 72% |
| **2-3** | 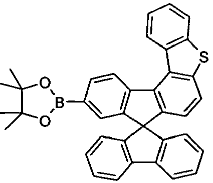 | 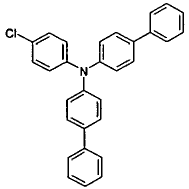 | 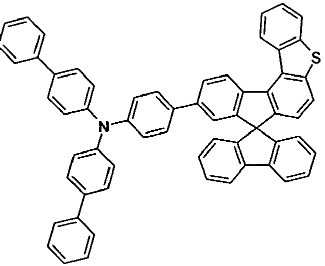 | 71% |
| **2-4** | 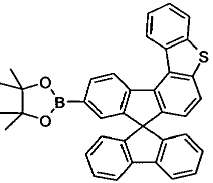 | 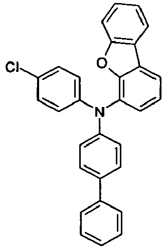 | 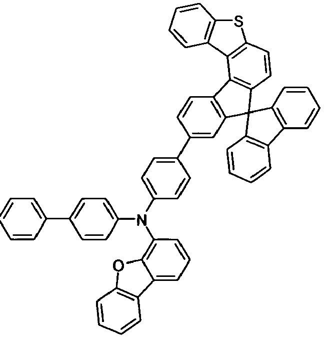 | 82% |
| **2-5** | 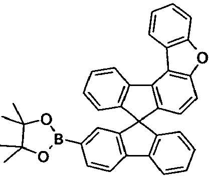 | 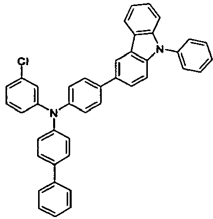 | 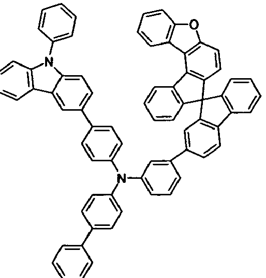 | 89% |
| **2-6** | 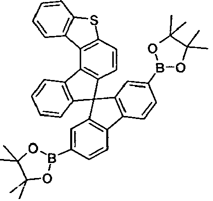 | 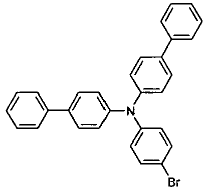 | 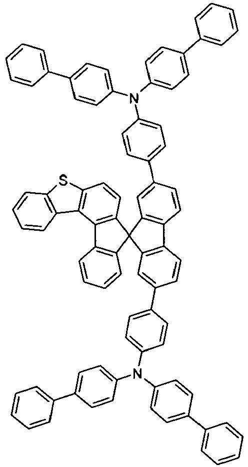 | 69% |
| **2-7** | 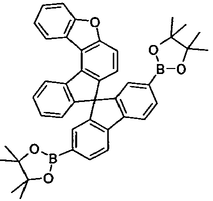 | 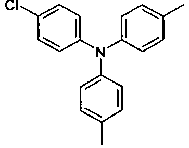 | 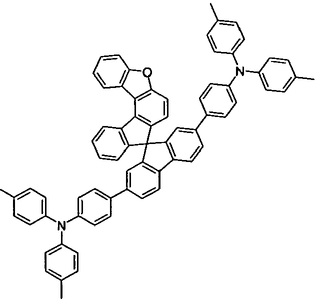 | 55% |
| **2-8** | 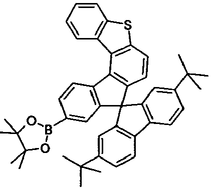 | 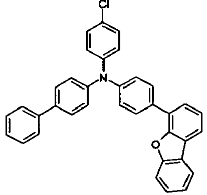 | 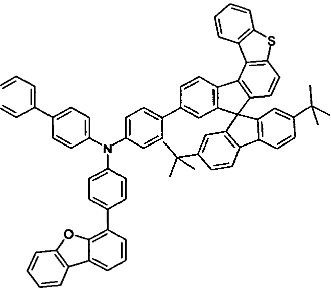 | 72% |
| **2-9** | 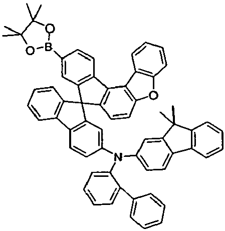 | 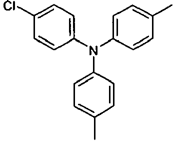 | 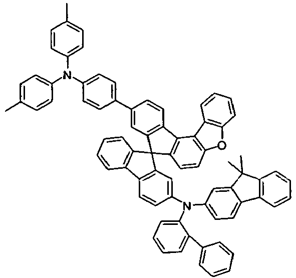 | 76% |

### Beispiel 3: Synthese der Verbindungen 3-1 bis 3-4

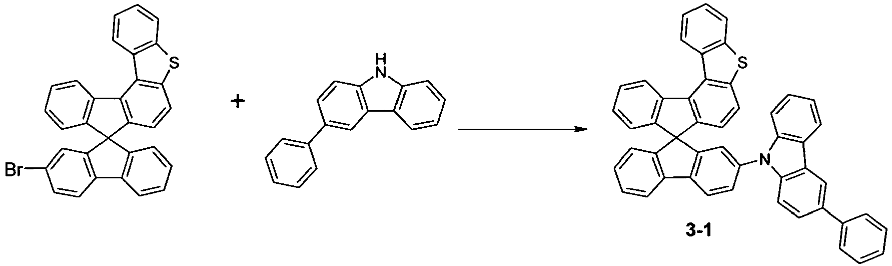

12,2 g (50 mmol) 3-Phenylcarbazol und 21 g (42 mmol) des Bromo-Spiroderivats werden in 300 mL Toluol gelöst. Die Lösung wird entgast und mit N₂ gesättigt. Danach wird sie mit 1,68 mL (1,68 mmol) einer 1M Tri-tert-Butylphosphin Lösung und 770 mg (0,84 mmol) Pd₂(dba)₃ versetzt und anschließend werden 6,18 g Natrium-tert-butylat (63 mmol) zugegeben. Die Reaktionsmischung wird 26 h unter Schutzatmosphäre zum Sieden erhitzt. Das Gemisch wird im Anschluss zwischen Toluol und Wasser verteilt, die organische Phase dreimal mit Wasser gewaschen und über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9% (HPLC). Die Ausbeute von Verbindung (**3-1**) beträgt 13,5 g (58% der Theorie).

### Synthese der Verbindungen (3-2) bis (3-4)

Analog zu der in Beispiel 1 beschriebenen Synthese von Verbindung (**3-1**) werden auch die folgenden Verbindungen (**3-2**) bis (**3-4**) hergestellt:

| | Edukt 1 | Edukt 2 | Produkt | Ausbeute |
|---|---|---|---|---|
| **3-2** | 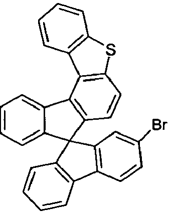 | 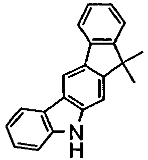 | 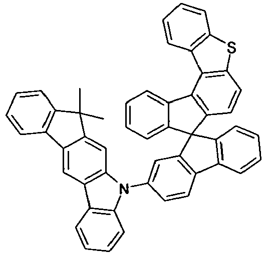 | 38% |
| | | 1257220-47-5 | | |
| **3-3** | 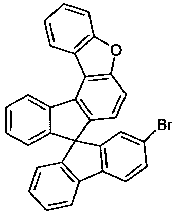 | 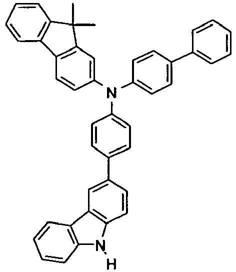 | 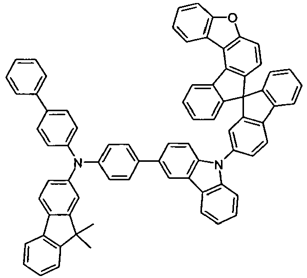 | 45% |
| | | 1427738-11-1 | | |
| **3-4** | 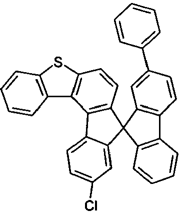 | 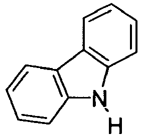 | 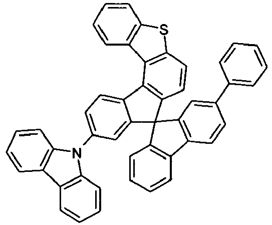 | 43% |

### B) Device-Beispiele

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (z.B. Materialien) angepasst wird.

In den folgenden erfindungsgemäßen Beispielen werden die Daten verschiedener OLEDs vorgestellt. Als Substrate werden Glasplättchen verwendet, die mit strukturiertem ITO (Indium Zinn Oxid) der Dicke 50nm beschichtet sind. Die OLEDs haben folgenden allgemeinen Schichtaufbau: Substrat / p-dotierte Lochtransportschicht (HIL) / Lochtransportschicht (HTL) / Elektronenblockierschicht (EBL) / Emissionsschicht (EML) / Elektronentransportschicht (ETL) / Elektroneninjektionsschicht (EIL) und abschließend eine Kathode. Die Kathode wird durch eine 100nm dicke Aluminiumschicht gebildet. Die zur Herstellung der OLEDs benötigten Materialien sind in Tabelle 1 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB(5%) bedeutet hierbei, dass das Material H1 in einem Volumenanteil von 95% und SEB in einem Volumenanteil von 5% in der Schicht vorliegt. Analog können auch die Elektronentransportschichten oder die Lochinjektionsschichten aus einer Mischung von zwei oder mehr Materialien bestehen. Die Zahl in Klammern hinter den Materialien gibt die jeweilige Schichtdicke an, in der die vorgenannten Materialien enthalten sind.

Die OLEDs werden standardmäßig charakterisiert. Hierfür wird die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Angabe EQE @ 10mA/cm² bezeichnet die externe Quanteneffizienz bei einer Stromdichte von 10mA/cm². LD80 @ 60mA/cm² ist die Lebensdauer, bis zu der die OLED bei einer Starthelligkeit bei konstantem Strom von 60mA/cm² auf 80% der Anfangsintensität abgefallen ist.

| ***Tabelle 1: Strukturen der verwendeten Materialien*** | | |
|---|---|---|
| 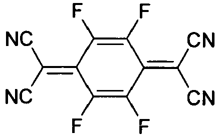 | 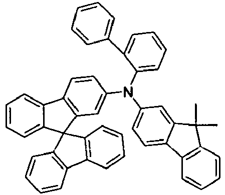 | 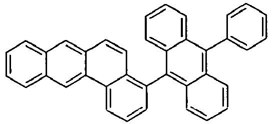 |
| F4TCNQ | HIM | H1 |
| 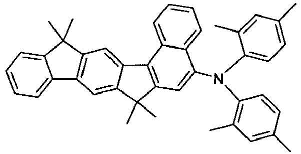 | 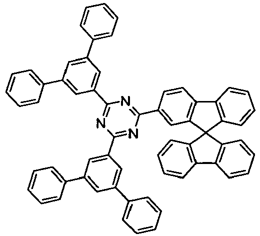 | 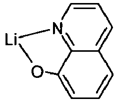 |
| SEB | ETM | LiQ |
| 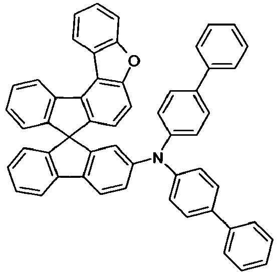 | 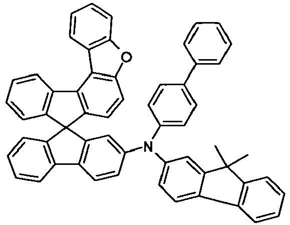 | 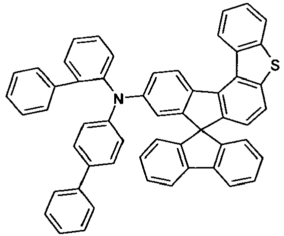 |
| HTM1 | HTM2 | HTM3 |
| 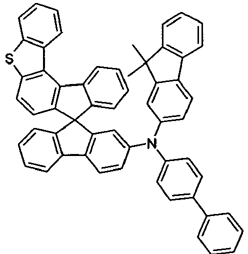 | 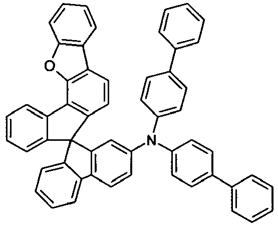 | 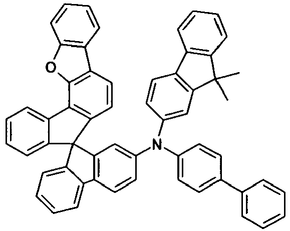 |
| HTM4 | HTMv1 | HTMv2 |
| 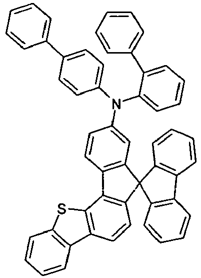 | 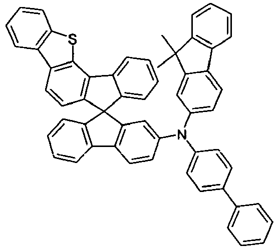 | |
| HTMv3 | HTMv4 | |

### Beispiel 1

Es wird die erfindungsgemäße Verbindung HTM1 mit der Vergleichsverbindung HTMv1 in einem blauen Stack miteinander verglichen. Der Aufbau des Stacks gliedert sich wie folgt: HIM:F4TCNQ(5%)(20nm) / HIM(175nm) / HTM1(20nm) / H1:SEB(5%)(20nm) / ETM:LiQ(50%)(30nm) / LiQ(1nm). Im Vergleichsbeispiel wird statt HTM1 in der betreffenden Schicht HTMv1 verdampft.
Die Evaluation der externen Quanteneffizienzen bei 10mA/cm² zeigt für die durchgeführten Experimente die folgenden Ergebnisse: HTM1 erzielt 8,1% EQE, wohingegen HTMv1 nur 6,6% erreicht.
Auch die Betriebsspannung der OLED mit erfindungsgemäßem Material liegt mit 3,86V deutlich unter der Spannung, die beim Vergleichsmaterial bei 10mA/cm² anliegt. Diese beträgt im Vergleichsfall 4,08V und ist demnach 6% höher.

### Beispiel 2

Ein weiteres erfindungsgemäßes Material HTM2 wird mit dem direkten Analogen HTMv2 mit gedrehter Dibenzofuraneinheit verglichen. Das OLED-Bauteil hat dabei die folgende Architektur: HIM:F4TCNQ(5%)(20nm) / HIM(175nm) / HTM2(20nm) bzw. HTMv2 (20nm) / H1:SEB(5%)(20nm) / ETM:LiQ(50%)(30nm) / LiQ(1nm).
Auch hier zeigt sich der Vorteil der erfindungsgemäßen Verbindung. Die externe Quanteneffizienz beträgt bei der Probe enthaltend HTM2 7,5%, wohingegen die Vergleichsprobe nur auf 7,2% EQE bei einer Stromdichte von 10mA/cm² kommt.

### Beispiel 3

Ein weiteres Bauteil wird mit dem Schichtaufbau HIM:F4TCNQ(5%)(20nm) / HIM(175nm) / HTM3(20nm) / H1:SEB(5%)(20nm) / ETM:LiQ(50%)(30nm) / LiQ(1nm) hergestellt. Im Vergleichsexperiment wird HTM3 durch HTMv3 ersetzt. Das Bauteil mit der erfindungsgemäßen Substanz in der EBL erzielt eine externe Quanteneffizienz bei 10mA/cm² von 7,3%. Das Bauteil mit der Vergleichssubstanz in der gleichen Funktionsschicht erreicht nur 7,0%.

### Beispiel 4

Schließlich werden noch die Verbindungen HTM4 und HTMv4 in einem Singulett Blau Stack getestet: HIM:F4TCNQ(5%)(20nm) / HIM(175nm) / HTM4(20nm) / H1:SEB(5%)(20nm) / ETM:LiQ(50%)(30nm) / LiQ(1nm). Im Vergleichstest wird statt HTM4 HTMv4 in die EBL eingebracht. Die erfindungsgemäße Substanz zeigt eine externe Quanteneffizienz bei 10mA/cm² von 7,5%. Die Vergleichsverbindung erreicht lediglich 7,2% EQE bei 10mA/cm².

## Patentansprüche

1. Verbindung der Formel (I) 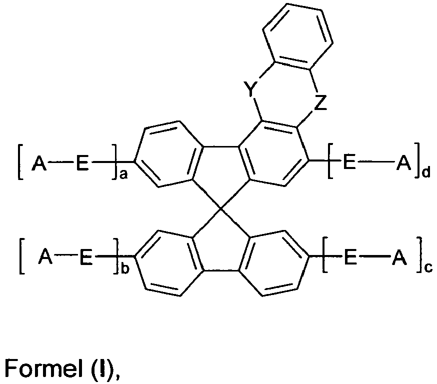 die an einer oder mehreren unsubstituiert dargestellten Positionen an der Grundstruktur gemäß Formel (I) mit je einem Rest R¹ substituiert sein kann; und
die die folgenden Definitionen der Variablen aufweist:
Y ist gewählt aus einer Einfachbindung, O, S oder Se;
Z ist gewählt aus O, S oder Se;
E ist bei jedem Auftreten gleich oder verschieden eine Einfachbindung oder ein aromatisches oder heteroaromatisches Ringsystem mit 6 bis 30 aromatischen Ringatomen, das mit einem oder mehreren Resten R² substituiert sein kann;
A ist bei jedem Auftreten gleich oder verschieden eine Gruppe der Formel (A1), (A2) oder (A3), die über die mit # markierte Bindung gebunden ist; 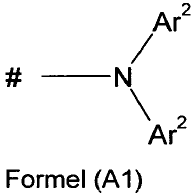 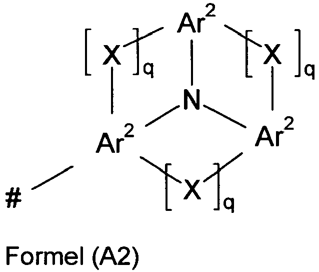 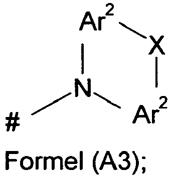
Ar² ist bei jedem Auftreten gleich oder verschieden ein aromatisches oder heteroaromatisches Ringsystem mit 6 bis 30 aromatischen Ringatomen, das mit einem oder mehreren Resten R² substituiert sein kann;
X ist bei jedem Auftreten gleich oder verschieden eine Einfachbindung oder eine Gruppe gewählt aus BR², C(R²)₂, Si(R²)₂, C=O, O, S, S=O, SO₂, NR², PR² oder P(=O)R²;
R¹ ist bei jedem Auftreten gleich oder verschieden gewählt aus H, D, F, C(=O)R³, CN, Si(R³)₃, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können;
R² ist bei jedem Auftreten gleich oder verschieden gewählt aus H, D, F, C(=O)R³, CN, Si(R³)₃, N(R³)₂, P(=O)(R³)₂, OR³, S(=O)R³, S(=O)₂R³, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R³ substituiert sein können; und wobei eine oder mehrere CH₂-Gruppen in den genannten Alkyl-, Alkoxy-, Alkenyl- und Alkinylgruppen durch -R³C=CR³-, -C≡C-, Si(R³)₂, C=O, C=NR³, -C(=O)O-, -C(=O)NR³-, NR³, P(=O)(R³), -O-, -S-, SO oder SO₂ ersetzt sein können;
R³ ist bei jedem Auftreten gleich oder verschieden gewählt aus H, D, F, C(=O)R⁴, CN, Si(R⁴)₃, N(R⁴)₂, P(=O)(R⁴)₂, OR⁴, S(=O)R⁴, S(=O)₂R⁴, geradkettigen Alkyl- oder Alkoxygruppen mit 1 bis 20 C-Atomen, verzweigten oder cyclischen Alkyl- oder Alkoxygruppen mit 3 bis 20 C-Atomen, Alkenyl- oder Alkinylgruppen mit 2 bis 20 C-Atomen, aromatischen Ringsystemen mit 6 bis 40 aromatischen Ringatomen, und heteroaromatischen Ringsystemen mit 5 bis 40 aromatischen Ringatomen; wobei zwei oder mehr Reste R¹ bzw. R² miteinander verknüpft sein können und einen Ring bilden können; wobei die genannten Alkyl-, Alkoxy-, Alkenyl- und Alkinylgruppen und die genannten aromatischen Ringsysteme und heteroaromatischen Ringsysteme jeweils mit einem oder mehreren Resten R⁴ substituiert sein können; und wobei eine oder mehrere CH₂-Gruppen in den genannten Alkyl-, Alkoxy-, Alkenyl- und Alkinylgruppen durch -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO oder SO₂ ersetzt sein können;
R⁴ ist bei jedem Auftreten gleich oder verschieden gewählt aus H, D, F, CN, Alkylgruppen mit 1 bis 20 C-Atomen, aromatischen Ringsystemen mit 6 bis 40 aromatischen Ringatomen und heteroaromatischen Ringsystemen mit 5 bis 40 aromatischen Ringatomen; wobei zwei oder mehr Reste R⁴ miteinander verknüpft sein können und einen Ring bilden können; und wobei die genannten Alkylgruppen, aromatischen Ringsysteme und heteroaromatischen Ringsysteme mit F oder CN substituiert sein können;
q ist bei jedem Auftreten gleich oder verschieden 0 oder 1, wobei mindestens ein q in Formel (A2) gleich 1 ist;
a, b, c und d sind bei jedem Auftreten gleich oder verschieden 0 oder 1, wobei mindestens einer der Indices a, b, c und d gleich 1 ist, und wobei für den Fall, dass einer oder mehrere der Indices a, b, c und d gleich 0 sind, an der betreffenden Position eine Gruppe R¹ gebunden ist.

2. Verbindung nach Anspruch 1, **dadurch gekennzeichnet, dass** Y eine Einfachbindung ist, und dass Z gewählt ist aus O oder S.

3. Verbindung nach Anspruch 1, **dadurch gekennzeichnet, dass** Y gewählt ist aus O oder S, und dass Z gewählt ist aus O oder S.

4. Verbindung nach einem oder mehreren der Ansprüche 1 bis 3, **dadurch gekennzeichnet, dass** E bei jedem Auftreten gleich oder verschieden gewählt ist aus einer Einfachbindung oder einer divalenten Gruppe abgeleitet von wahlweise mit Resten R² substituiertem Benzol, Biphenyl, Terphenyl, Fluoren, Spirobifluoren, Indenofluoren, Carbazol, Dibenzofuran, Dibenzothiophen, oder einer Kombination von zwei oder mehr dieser Gruppen, wobei nicht mehr als 30 aromatische Ringatome in der Gruppe E vorliegen.

5. Verbindung nach einem oder mehreren der Ansprüche 1 bis 4, **dadurch gekennzeichnet, dass** X eine Einfachbindung ist.

6. Verbindung nach einem oder mehreren der Ansprüche 1 bis 5, **dadurch gekennzeichnet, dass** A bei jedem Auftreten gleich oder verschieden eine Gruppe der Formel (A-1) oder (A-3) ist.

7. Verbindung nach einem oder mehreren der Ansprüche 1 bis 6, **dadurch gekennzeichnet, dass** Ar² bei jedem Auftreten gleich oder verschieden gewählt ist aus Phenyl, Biphenyl, Terphenyl, Fluorenyl, Spirobifluorenyl, Indenofluorenyl, Naphthyl, Phenanthrenyl, Furanyl, Benzofuranyl, Dibenzofuranyl, Thiophenyl, Benzothiophenyl, Dibenzothiophenyl, Carbazolyl, Indolocarbazolyl und Indenocarbazolyl, welche jeweils mit einem oder mehreren Resten R² substituiert sein können.

8. Verbindung nach einem oder mehreren der Ansprüche 1 bis 7, **dadurch gekennzeichnet, dass** R¹ bei jedem Auftreten gleich oder verschieden gewählt ist aus H, F, CN, Methyl, tert-Butyl, Phenyl, Biphenyl, Dibenzofuran, Dibenzothiophen und Carbazol.

9. Verbindung nach einem oder mehreren der Ansprüche 1 bis 8, **dadurch gekennzeichnet, dass** R¹ gleich H ist.

10. Verbindung nach einem oder mehreren der Ansprüche 1 bis 9, **dadurch gekennzeichnet, dass** genau einer der Indices a, b, c und d gleich 1 ist und die anderen Indices gleich 0 sind; oder dass genau zwei der Indices a, b, c und d gleich 1 sind, und die anderen Indices gleich 0 sind.

11. Verbindung nach einem oder mehreren der Ansprüche 1 bis 10, **dadurch gekennzeichnet, dass** der Index d gleich 0 ist, und dass genau einer oder genau zwei der Indices a, b und c gleich 1 sind.

12. Verbindung nach einem oder mehreren der Ansprüche 1 bis 11, **dadurch gekennzeichnet, dass** sie der folgenden Ausführungsform von Formel (I) entspricht 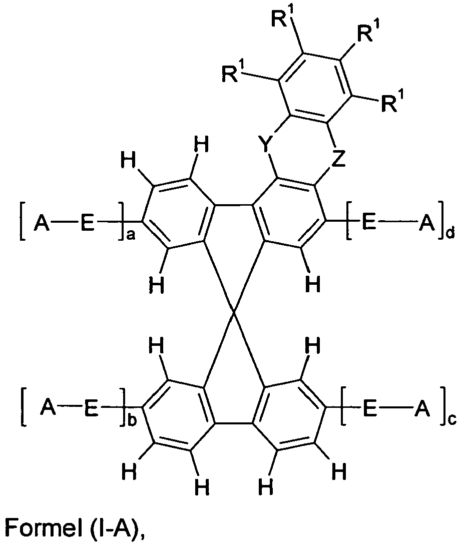 wobei die auftretenden Variablen definiert sind wie in einem oder mehreren der Ansprüche 1 bis 11.

13. Verfahren zur Herstellung einer Verbindung der Formel (I) nach einem oder mehreren der Ansprüche 1 bis 12, **dadurch gekennzeichnet, dass** zunächst das Spirobifluoren-Grundgerüst hergestellt wird, und in einem späteren Schritt über eine metallorganische Kupplungsreaktion eine Arylamino- oder eine Carbazolgruppe oder eine Aryl- oder Heteroarylgruppe, die mit einer Arylamino- oder Carbazolgruppe substituiert ist, eingeführt wird.

14. Oligomere, Polymere oder Dendrimere, enthaltend eine oder mehrere Verbindungen gemäß Formel (I) nach einem oder mehreren der Ansprüche 1 bis 12, wobei die Bindung(en) zum Polymer, Oligomer oder Dendrimer an beliebigen, in Formel (I) mit R¹ oder R² substituierten Positionen lokalisiert sein können.

15. Formulierung, enthaltend mindestens eine Verbindung nach einem oder mehreren der Ansprüche 1 bis 12, sowie mindestens ein Lösungsmittel.

16. Elektronische Vorrichtung, enthaltend mindestens eine Verbindung nach einem oder mehreren der Ansprüche 1 bis 12.

17. Elektronische Vorrichtung nach Anspruch 16, **dadurch gekennzeichnet, dass** sie eine organische Elektrolumineszenzvorrichtung ist, enthaltend Anode, Kathode und mindestens eine emittierende Schicht, wobei mindestens eine organische Schicht der Vorrichtung, die eine emittierende Schicht, eine Lochtransportschicht oder eine andere Schicht sein kann, die mindestens eine Verbindung enthält.

18. Elektronische Vorrichtung nach Anspruch 17, **dadurch gekennzeichnet, dass** die mindestens eine organische Schicht gewählt ist aus einer Lochtransportschicht, bevorzugt einer Elektronenblockierschicht, oder aus einer emittierenden Schicht, bevorzugt einer emittierenden Schicht, welche einen oder mehrere phosphoreszierende Emitter enthält.

19. Verwendung einer Verbindung nach einem oder mehreren der Ansprüche 1 bis 12 in einer elektronischen Vorrichtung.

## Claims

1. Compound of the formula (I) 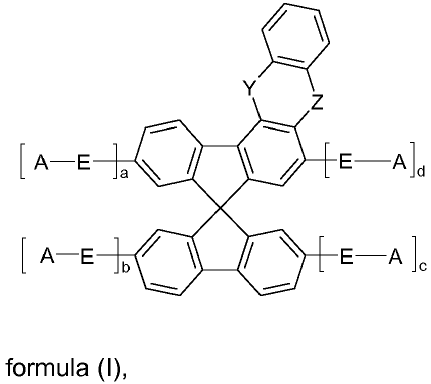 which may be substituted by a radical R¹ at each of one or more positions depicted as unsubstituted on the basic structure of the formula (I); and
which has the following definitions of the variables:
Y is selected from a single bond, O, S or Se;
Z is selected from O, S or Se;
E is on each occurrence, identically or differently, a single bond or an aromatic or heteroaromatic ring system having 6 to 30 aromatic ring atoms, which may be substituted by one or more radicals R²;
A is on each occurrence, identically or differently, a group of the formula (A1), (A2) or (A3), which is bonded via the bond marked by #; 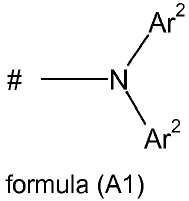 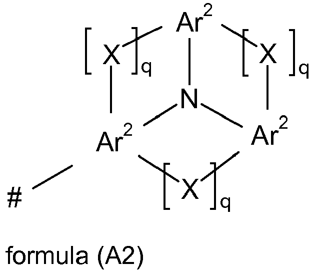 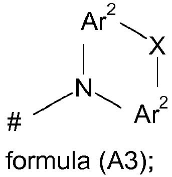
Ar² is on each occurrence, identically or differently, an aromatic or heteroaromatic ring system having 6 to 30 aromatic ring atoms, which may be substituted by one or more radicals R²;
X is on each occurrence, identically or differently, a single bond or a group selected from BR², C(R²)₂, Si(R²)₂, C=O, O, S, S=O, SO₂, NR², PR² or P(=O)R ²;
R¹ is selected on each occurrence, identically or differently, from H, D, F, C(=O)R³, CN, Si(R³)₃, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ may be linked to one another and may form a ring; where the said alkyl, alkoxy, alkenyl and alkynyl groups and the said aromatic ring systems and heteroaromatic ring systems may in each case be substituted by one or more radicals R³; and where one or more CH2 groups in the said alkyl, alkoxy, alkenyl and alkynyl groups may be replaced by -R³C=CR³-, -C≡C-, Si(R³)₂, C=O, C=NR³, -C(=O)O-, -C(=O)NR³-, NR³, P(=O)(R³), -O-, -S-, SO or SO₂;
R² is selected on each occurrence, identically or differently, from H, D, F, C(=O)R³, CN, Si(R³)₃, N(R³)₂, P(=O)(R³)₂, OR³, S(=O)R³, S(=O)₂R³, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R² may be linked to one another and may form a ring; where the said alkyl, alkoxy, alkenyl and alkynyl groups and the said aromatic ring systems and heteroaromatic ring systems may in each case be substituted by one or more radicals R³; and where one or more CH₂ groups in the said alkyl, alkoxy, alkenyl and alkynyl groups may be replaced by-R³C=CR³-, -C≡C-, Si(R³)₂, C=O, C=NR³, -C(=O)O-, -C(=O)NR³-, NR³, P(=O)(R³), -O-, -S-, SO or SO₂;
R³ is selected on each occurrence, identically or differently, from H, D, F, C(=O)R⁴, CN, Si(R⁴)₃, N(R⁴)₂, P(=O)(R⁴)₂, OR⁴, S(=O)R⁴, S(=O)₂R⁴, straight-chain alkyl or alkoxy groups having 1 to 20 C atoms, branched or cyclic alkyl or alkoxy groups having 3 to 20 C atoms, alkenyl or alkynyl groups having 2 to 20 C atoms, aromatic ring systems having 6 to 40 aromatic ring atoms, and heteroaromatic ring systems having 5 to 40 aromatic ring atoms; where two or more radicals R¹ or R² may be linked to one another and may form a ring; where the said alkyl, alkoxy, alkenyl and alkynyl groups and the said aromatic ring systems and heteroaromatic ring systems may in each case be substituted by one or more radicals R⁴; and where one or more CH2 groups in the said alkyl, alkoxy, alkenyl and alkynyl groups may be replaced by-R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO or SO₂;
R⁴ is selected on each occurrence, identically or differently, from H, D, F, CN, alkyl groups having 1 to 20 C atoms, aromatic ring systems having 6 to 40 aromatic ring atoms and heteroaromatic ring systems having 5 to 40 aromatic ring atoms; where two or more radicals R⁴ may be linked to one another and may form a ring; and where the said alkyl groups, aromatic ring systems and heteroaromatic ring systems may be substituted by F or CN;
q is on each occurrence, identically or differently, 0 or 1, where at least one q in formula (A2) is equal to 1;
a, b, c and d are on each occurrence, identically or differently, 0 or 1, where at least one of the indices a, b, c and d is equal to 1, and where, in the case where one or more of the indices a, b, c and d are equal to 0, a group R¹ is bonded at the position in question.

2. Compound according to Claim 1, **characterised in that** Y is a single bond, and **in that** Z is selected from O or S.

3. Compound according to Claim 1, **characterised in that** Y is selected from O or S, and **in that** Z is selected from O or S.

4. Compound according to one or more of Claims 1 to 3, **characterised in that** E is selected on each occurrence, identically or differently, from a single bond or a divalent group derived from benzene, biphenyl, terphenyl, fluorene, spirobifluorene, indenofluorene, carbazole, dibenzofuran, dibenzothiophene, each of which is optionally substituted by radicals R², or a combination of two or more of these groups, where not more than 30 aromatic ring atoms are present in the group E.

5. Compound according to one or more of Claims 1 to 4, **characterised in that** X is a single bond.

6. Compound according to one or more of Claims 1 to 5, **characterised in that** A is on each occurrence, identically or differently, a group of the formula (A-1) or (A-3).

7. Compound according to one or more of Claims 1 to 6, **characterised in that** Ar² is selected on each occurrence, identically or differently, from phenyl, biphenyl, terphenyl, fluorenyl, spirobifluorenyl, indenofluorenyl, naphthyl, phenanthrenyl, furanyl, benzofuranyl, dibenzofuranyl, thiophenyl, benzothiophenyl, dibenzothiophenyl, carbazolyl, indolocarbazolyl and indenocarbazolyl, each of which may be substituted by one or more radicals R².

8. Compound according to one or more of Claims 1 to 7, **characterised in that** R¹ is selected on each occurrence, identically or differently, from H, F, CN, methyl, tert-butyl, phenyl, biphenyl, dibenzofuran, dibenzothiophene and carbazole.

9. Compound according to one or more of Claims 1 to 8, **characterised in that** R¹ is equal to H.

10. Compound according to one or more of Claims 1 to 9, **characterised in that** precisely one of the indices a, b, c and d is equal to 1 and the other indices are equal to 0; or **in that** precisely two of the indices a, b, c and d are equal to 1 and the other indices are equal to 0.

11. Compound according to one or more of Claims 1 to 10, **characterised in that** the index d is equal to 0, and **in that** precisely one or precisely two of the indices a, b and c are equal to 1.

12. Compound according to one or more of Claims 1 to 11, **characterised in that** it corresponds to the following embodiment of the formula (I) 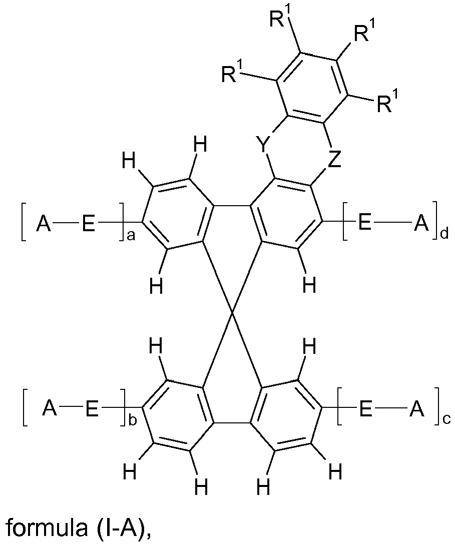 where the variables occurring are defined as in one or more of Claims 1 to 11.

13. Process for the preparation of a compound of the formula (I) according to one or more of Claims 1 to 12, **characterised in that** firstly the spirobifluorene skeleton is produced, and in a later step an arylamino or carbazole group or an aryl or heteroaryl group which is substituted by an arylamino or carbazole group is introduced via an organometallic coupling reaction.

14. Oligomers, polymers or dendrimers containing one or more compounds of the formula (I) according to one or more of Claims 1 to 12, where the bond(s) to the polymer, oligomer or dendrimer may be localised at any desired positions in formula (I) that are substituted by R¹ or R².

15. Formulation comprising at least one compound according to one or more of Claims 1 to 12 and at least one solvent.

16. Electronic device containing at least one compound according to one or more of Claims 1 to 12.

17. Electronic device according to Claim 16, **characterised in that** it is an organic electroluminescent device containing anode, cathode and at least one emitting layer, where at least one organic layer of the device, which may be an emitting layer, a hole-transport layer or another layer, comprises the at least one compound.

18. Electronic device according to Claim 17, **characterised in that** the at least one organic layer is selected from a hole-transport layer preferably an electron-blocking layer, or from an emitting layer, preferably an emitting layer which comprises one or more phosphorescent emitters.

19. Use of a compound according to one or more of Claims 1 to 12 in an electronic device.

## Revendications

1. Composé de la formule (I) : 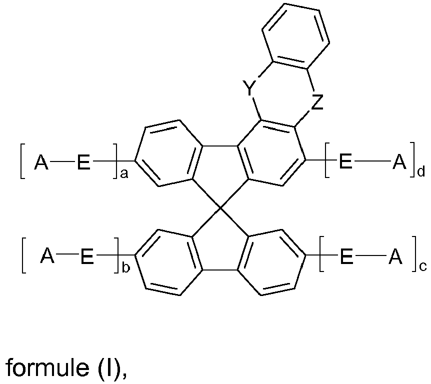 lequel peut être substitué par un radical R¹ au niveau de chacune d'une ou de plusieurs position(s) qui est/sont représentée(s) comme étant non substituée(s) sur la structure de base de la formule (I) ; et
lequel présente les définitions qui suivent des variables :
Y est sélectionné parmi une liaison simple, O, S ou Se ;
Z est sélectionné parmi O, S ou Se ;
E est pour chaque occurrence, de manière identique ou différente, une liaison simple ou un système de cycle aromatique ou hétéroaromatique qui comporte de 6 à 30 atomes de cycle aromatique, lequel peut être substitué par un radical ou par plusieurs radicaux R²;
A est pour chaque occurrence, de manière identique ou différente, un groupe de la formule (A1), (A2) ou (A3), lequel est lié via la liaison qui est indiquée au moyen de # ; 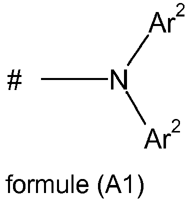 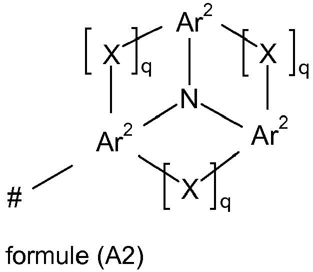 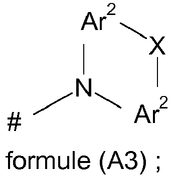
Ar² est pour chaque occurrence, de manière identique ou différente, un système de cycle aromatique ou hétéroaromatique qui comporte de 6 à 30 atomes de cycle aromatique, lequel peut être substitué par un radical ou par plusieurs radicaux R²;
X est pour chaque occurrence, de manière identique ou différente, une liaison simple ou un groupe qui est sélectionné parmi BR², C(R²)₂, Si(R²)₂, C=O, O, S, S=O, SO₂, NR², PR² ou P(=O)R² ;
R¹ est sélectionné pour chaque occurrence, de manière identique ou différente, parmi H, D, F, C(=O)R³, CN, Si(R³)₃, P(=O)(R³)₂, OR³, S(=O)R³, S(=O)₂R³, des groupes alkyle ou alcoxy en chaîne droite qui comportent de 1 à 20 atome(s) de C, des groupes alkyle ou alcoxy ramifiés ou cycliques qui comportent de 3 à 20 atomes de C, des groupes alkényle ou alkynyle qui comportent de 2 à 20 atomes de C, des systèmes de cycle aromatique qui comportent de 6 à 40 atomes de cycle aromatique, et des systèmes de cycle hétéroaromatique qui comportent de 5 à 40 atomes de cycle aromatique ; où deux radicaux R¹ ou plus peuvent être liés l'un à l'autre ou les uns aux autres et peuvent former un cycle ; où lesdits groupes alkyle, alcoxy, alkényle et alkynyle et lesdits systèmes de cycle aromatique et lesdits systèmes de cycle hétéroaromatique peuvent dans chaque cas être substitués par un radical ou par plusieurs radicaux R³; et où un ou plusieurs groupe(s) CH2 dans lesdits groupes alkyle, alcoxy, alkényle et alkynyle peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂;
R² est sélectionné pour chaque occurrence, de manière identique ou différente, parmi H, D, F, C(=O)R³, CN, Si(R³)₃, N(R³)₂, P(=O)(R³)₂, OR³, S(=O)R³, S(=O)₂R³, des groupes alkyle ou alcoxy en chaîne droite qui comportent de 1 à 20 atome(s) de C, des groupes alkyle ou alcoxy ramifiés ou cycliques qui comportent de 3 à 20 atomes de C, des groupes alkényle ou alkynyle qui comportent de 2 à 20 atomes de C, des systèmes de cycle aromatique qui comportent de 6 à 40 atomes de cycle aromatique, et des systèmes de cycle hétéroaromatique qui comportent de 5 à 40 atomes de cycle aromatique ; où deux radicaux R² ou plus peuvent être liés l'un à l'autre ou les uns aux autres et peuvent former un cycle ; où lesdits groupes alkyle, alcoxy, alkényle et alkynyle et lesdits systèmes de cycle aromatique et lesdits systèmes de cycle hétéroaromatique peuvent dans chaque cas être substitués par un radical ou par plusieurs radicaux R³; et où un ou plusieurs groupe(s) CH2 dans lesdits groupes alkyle, alcoxy, alkényle et alkynyle peut/peuvent être remplacé(s) par -R³C=CR³-, -C≡C-, Si(R³)₂, C=O, C=NR³, -C(=O)O-, -C(=O)NR³-, NR³, P(=O)(R³), -O-, -S-, SO ou SO₂;
R³ est sélectionné pour chaque occurrence, de manière identique ou différente, parmi H, D, F, C(=O)R⁴, CN, Si(R⁴)₃, N(R⁴)₂, P(=O)(R⁴)₂, OR⁴, S(=O)R⁴, S(=O)₂R⁴, des groupes alkyle ou alcoxy en chaîne droite qui comportent de 1 à 20 atome(s) de C, des groupes alkyle ou alcoxy ramifiés ou cycliques qui comportent de 3 à 20 atomes de C, des groupes alkényle ou alkynyle qui comportent de 2 à 20 atomes de C, des systèmes aromatiques qui comportent de 6 à 40 atomes de cycle aromatique, et des systèmes de cycle hétéroaromatique qui comportent de 5 à 40 atomes de cycle aromatique ; où deux radicaux R¹ ou R² ou plus peuvent être liés l'un à l'autre ou les uns aux autres et peuvent former un cycle ; où lesdits groupes alkyle, alcoxy, alkényle et alkynyle et lesdits systèmes de cycle aromatique et lesdits systèmes de cycle hétéroaromatique peuvent dans chaque cas être substitués par un radical ou par plusieurs radicaux R⁴; et où un ou plusieurs groupe(s) CH2 dans lesdits groupes alkyle, alcoxy, alkényle et alkynyle peut/peuvent être remplacé(s) par -R⁴C=CR⁴-, -C≡C-, Si(R⁴)₂, C=O, C=NR⁴, -C(=O)O-, -C(=O)NR⁴-, NR⁴, P(=O)(R⁴), -O-, -S-, SO ou SO₂;
R⁴ est sélectionné pour chaque occurrence, de manière identique ou différente, parmi H, D, F, CN, des groupes alkyle qui comportent de 1 à 20 atome(s) de C, des systèmes de cycle aromatique qui comportent de 6 à 40 atomes de cycle aromatique et des systèmes de cycle hétéroaromatique qui comportent de 5 à 40 atomes de cycle aromatique ; où deux radicaux R⁴ ou plus peuvent être liés l'un à l'autre ou les uns aux autres et peuvent former un cycle ; et où lesdits groupes alkyle, lesdits systèmes de cycle aromatique et lesdits systèmes de cycle hétéroaromatique peuvent être substitués par F ou CN ;
q est pour chaque occurrence, de manière identique ou différente, 0 ou 1, où au moins un q dans la formule (A2) est égal à 1 ;
a, b, c et d sont pour chaque occurrence, de manière identique ou différente, 0 ou 1, où au moins l'un des indices a, b, c et d est égal à 1, et où, dans le cas dans lequel un ou plusieurs des indices a, b, c et d est égal/sont égaux à 0, un groupe R¹ est lié au niveau de la position en question.

2. Composé selon la revendication 1, **caractérisé en ce que** Y est une liaison simple, et **en ce que** Z est sélectionné parmi O ou S.

3. Composé selon la revendication 1, **caractérisé en ce que** Y est sélectionné parmi O ou S, et **en ce que** Z est sélectionné parmi O ou S.

4. Composé selon une ou plusieurs des revendications 1 à 3, **caractérisé en ce que** E est sélectionné pour chaque occurrence, de manière identique ou différente, parmi une liaison simple ou un groupe divalent qui est dérivé à partir du benzène, du biphényle, du terphényle, du fluorène, du spirobifluorène, de l'indénofluorène, du carbazole, du dibenzofurane, du dibenzothiophène, dont chacun est en option substitué par des radicaux R², ou une combinaison de deux ou plus de ces groupes, où pas plus de 30 atomes de cycle aromatique sont présents dans le groupe E.

5. Composé selon une ou plusieurs des revendications 1 à 4, **caractérisé en ce que** X est une liaison simple.

6. Composé selon une ou plusieurs des revendications 1 à 5, **caractérisé en ce que** A est pour chaque occurrence, de manière identique ou différente, un groupe de la formule (A-1) ou (A-3).

7. Composé selon une ou plusieurs des revendications 1 à 6, **caractérisé en ce que** Ar² est sélectionné pour chaque occurrence, de manière identique ou différente, parmi phényle, biphényle, terphényle, fluorényle, spirobifluorényle, indénofluorényle, naphtyle, phénanthrényle, furanyle, benzofuranyle, dibenzofuranyle, thiophényle, benzothiophényle, dibenzothiophényle, carbazolyle, indolocarbazolyle et indénocarbazolyle, dont chacun peut être substitué par un radical ou par plusieurs radicaux R².

8. Composé selon une ou plusieurs des revendications 1 à 7, **caractérisé en ce que** R¹ est sélectionné pour chaque occurrence, de manière identique ou différente, parmi H, F, CN, méthyle, tert-butyle, phényle, biphényle, dibenzofurane, dibenzothiophène et carbazole.

9. Composé selon une ou plusieurs des revendications 1 à 8, **caractérisé en ce que** R¹ est égal à H.

10. Composé selon une ou plusieurs des revendications 1 à 9, **caractérisé en ce que** précisément l'un des indices a, b, c et d est égal à 1 et les autres indices sont égaux à 0 ; ou **en ce que** précisément deux des indices a, b, c et d sont égaux à 1 et les autres indices sont égaux à 0.

11. Composé selon une ou plusieurs des revendications 1 à 10, **caractérisé en ce que** l'indice d est égal à 0, et **en ce que** précisément un ou précisément deux des indices a, b et c est égal/sont égaux à 1.

12. Composé selon une ou plusieurs des revendications 1 à 11, **caractérisé en ce qu'**il correspond au mode de réalisation qui suit de la formule (I) : 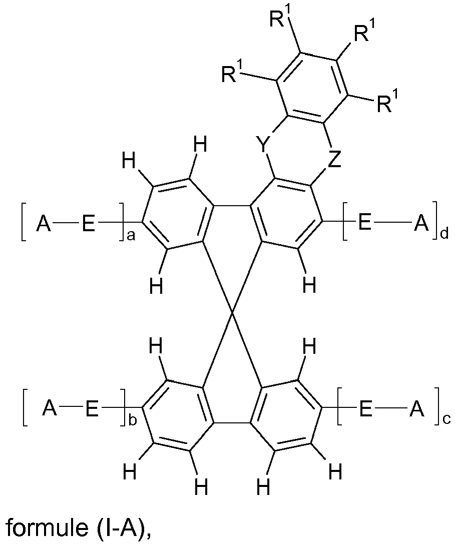 dans laquelle les variables qui sont rencontrées sont définies tel que selon une ou plusieurs des revendications 1 à 11.

13. Procédé pour la préparation d'un composé de la formule (I) selon une ou plusieurs des revendications 1 à 12, **caractérisé en ce que**, en premier lieu, le squelette du spirobifluorène est produit, et au niveau d'une étape ultérieure, un groupe arylamino ou carbazole ou un groupe aryle ou hétéroaryle qui est substitué par un groupe arylamino ou carbazole est introduit via une réaction de couplage organométallique.

14. Oligomères, polymères ou dendrimères contenant un ou plusieurs composé(s) de la formule (I) selon une ou plusieurs des revendications 1 à 12, où la/les liaison(s) sur le polymère, l'oligomère ou le dendrimère peut/peuvent être localisée(s) au niveau de n'importe quelles positions souhaitées dans la formule (I) qui sont substituées par R¹ ou par R².

15. Formulation comprenant au moins un composé selon une ou plusieurs des revendications 1 à 12 et au moins un solvant.

16. Dispositif électronique contenant au moins un composé selon une ou plusieurs des revendications 1 à 12.

17. Dispositif électronique selon la revendication 16, **caractérisé en ce qu'**il s'agit d'un dispositif électroluminescent organique qui contient une anode, une cathode et au moins une couche d'émission, où au moins une couche organique du dispositif, qui peut être une couche d'émission, une couche de transport de trous ou une autre couche, comprend l'au moins un composé.

18. Dispositif électronique selon la revendication 17, **caractérisé en ce que** l'au moins une couche organique est sélectionnée parmi une couche de transport de trous, de préférence une couche de blocage d'électrons, ou parmi une couche d'émission, de préférence une couche d'émission qui comprend un ou plusieurs émetteur(s), phosphorescent(s).

19. Utilisation d'un composé selon une ou plusieurs des revendications 1 à 12 dans un dispositif électronique.
